(19) Europäisches Patentamt
European Patent Office
Office européen des brevets

(11) **EP 2 291 652 B1**

(12) **EUROPEAN PATENT SPECIFICATION**

(45) Date of publication and mention
of the grant of the patent:
**11.09.2013   Bulletin 2013/37**

(51) Int Cl.:
*G01N 33/50* (2006.01)      *A01N 1/02* (2006.01)

(21) Application number: **09742400.6**

(22) Date of filing: **08.05.2009**

(86) International application number:
**PCT/GB2009/050482**

(87) International publication number:
**WO 2009/136204 (12.11.2009 Gazette 2009/46)**

(54) **STABILISATION OF BLOOD CELL CONJUGATES**

STABILISIERUNG VON BLUTZELLKONJUGATEN

STABILISATION DE CONJUGUÉS DE GLOBULES

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority:  **09.05.2008   GB 0808413**

(43) Date of publication of application:
**09.03.2011   Bulletin 2011/10**

(73) Proprietor: **The University of Nottingham
Nottingham Nottinghamshire NG7 2RD (GB)**

(72) Inventors:
• **FOX, Susan Carol
Selston
Nottingham NG7 2UH (GB)**
• **HEPTINSTALL, Stanley
Nottingham
Nottinghamshire NG7 2UH (GB)**

• **MAY, Jane Alison
Nottingham
Nottinghamshire NG7 2UH (GB)**

(74) Representative: **Atkinson, Jennifer et al
Barker Brettell LLP
100 Hagley Road
Edgbaston
Birmingham
B16 8QQ (GB)**

(56) References cited:
**US-A- 3 925 541        US-A- 5 503 982
US-A1- 2004 038 424    US-A1- 2007 166 389**

• **SCHMIDT VOLKER ET AL: "ThromboFix (TM)
Platelet Stabilizer: Advances in clinical platelet
analyses by flow cytometry?", PLATELETS,
TAYLOR AND FRANCIS GROUP, EDINBURGH,
vol. 17, no. 4, 1 June 2006 (2006-06-01), pages
266-273, XP008108523, ISSN: 0953-7104, DOI:
10.1080/09537100500197772**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to methods and kits for stabilising blood cell conjugates in blood samples for subsequent analysis. The invention particularly relates to methods and kits for use in fixing and stabilising platelet aggregates in blood samples, and methods for detecting the degree of platelet aggregation in a sample. The invention extends to kits for monitoring the efficacy of anti-thrombotic treatment regimes using platelet aggregation analysis.

Blood cells are known to interact with each other and form cell-cell conjugates. Potentially, erythrocytes (ie red blood cells) may form conjugates with platelets, or with leukocytes (ie white blood cells), such as monocytes, neutrophils or lymphocytes. Furthermore, leukocytes may form conjugates with other leukocytes or platelets. Platelets are very sticky cells and it is well recognized that, in addition to sticking to each other to form aggregates, they form conjugates with other cell types, such as erythrocytes and, in particular with leukocytes. There is a hierarchical tendency of activated platelets to bind primarily to monocytes, to neutrophils to a lesser extent, and to lymphocytes to a still lesser extent.

Platelets are essential cells involved in the cessation of bleeding following tissue trauma and vascular injury. Their role in these processes is central to haemostasis. Platelets circulate within the blood vessels alongside other blood cells, such as erythrocytes and leukocytes. Platelets are anucleate, disc-shaped cells which, following injury, rapidly become sticky when activated and release substances that cause them to aggregate together at the site of injury to form a platelet plug. These platelet plugs act to arrest bleeding and ultimately interact with coagulation proteins such as fibrinogen to form a stable blood clot.

Many investigations over the last 40 years have identified the processes and substances involved in the aggregation of platelets. Platelets are activated and aggregate following exposure to stimulants, such as collagen, adenosine diphosphate (ADP), Platelet Activating Factor (PAF), 5-hydroxytryptamine (5-HT), epinephrine and thrombin. When platelet stimulants are added to blood, the platelets become activated and express glycoprotein IIb/IIIa on their surface. If the platelets are in close contact, and agitated sufficiently, the glycoprotein IIb/IIIa sites on the platelet surface link the platelets together via fibrinogen bridges to form platelet aggregates.

Platelets also play a key role in pathological processes associated with thrombosis. These include cardiovascular events, such as angina and myocardial infarction, and cerebrovascular events, such as transient ischaemic attack and stroke. Anti-thrombotic therapies aim to reduce platelet activation sufficiently to inhibit aggregation and thrombus formation, but not cause excessive bleeding. The efficacy of anti-thrombotic therapies may be assessed by measuring the expression of platelet activation markers, such as CD62P, in the patient's blood. WO 2008/107724 describes compositions for use in stabilising the expression of cell markers (such as cell activation markers) present on blood cells, such as platelets. In order to assess the effects of anti-thrombotic therapies or platelet stimulants on expression of the platelet activation marker CD62P, it is necessary to treat the blood samples and avoid excessive agitation in order to limit cell-cell contact and prevent platelet aggregation from taking place. Typically, ethylenediamine tetraacetic acid (EDTA) is used to inhibit the formulation of platelet aggregates and other blood cell conjugates.

The efficacy of anti-thrombotic therapy may alternatively be monitored by assessing platelet aggregation responses, and this measure is often considered to be more acceptable or better recognised in the clinical arena. Indeed, the efficacy of anti-thrombotic therapy has been monitored for many years by measuring platelet aggregation responses. These were originally performed in the 1960-70s using optical instruments which measured aggregation in stirred samples of platelet-rich plasma (PRP) incubated at 37°C. PRP was prepared from anti-coagulated blood which was centrifuged in tubes by a slow centrifugation process. This process results in a top layer of platelets suspended in plasma, while leukocytes and erythrocytes sediment to the bottom of the tube. An aliquot of PRP was placed in a tube in an aggregometer, which optically monitored changes in the opacity of the PRP. This changed from cloudy to clear when the platelets were activated, became sticky and aggregated together. Optical aggregometry is still used today to measure platelet aggregation. However, there are limitations in its use including the need for good laboratory facilities to prepare platelet rich plasma, and large volumes of blood from which to prepare it.

In the 1980s, platelet counters were developed which could electronically measure the number of platelets in a sample of whole blood. These counters worked by measuring changes in impedance across two electrodes as the blood cells passed through a small aperture in a flow chamber. The signals generated were then computed to calculate platelet count. Techniques were developed to activate and stir whole blood samples at 37 °C and make use of these counters to monitor platelet aggregation in anti-coagulated whole blood. Incubators, which incorporated a magnetic stirrer, were developed in order to standardise temperature and stirring conditions for whole blood platelet aggregation. Aggregation was monitored as a fall in the number of single platelets in the blood samples. A further advance was made when it was found that any platelet aggregates that had formed during activation and stirring could be fixed using a formaldehyde solution so that they could be counted later at the end of the experiment. The use of this solution fixed the aggregates and allowed samples to be kept for up to two hours.

In the 1990s, flow cytometers were developed, and this provided further advances in the study of platelet behaviour. In a flow cytometer, a beam of laser light is directed at a stream of hydrodynamically focused cells in a flow chamber. A detector monitors and records the scatter of the light as each cell passes in a single file. This is recorded as forward scatter (an indication of size) and side-scatter (an indication of granularity) for each cell. Each cell that passes the laser is recorded as an event. Other detectors monitor changes in the fluorescence of the cells to which specific antibody-conjugated fluorochromes have been previously attached. All of the information collected from the flow cytometer is analysed by computer to produce dot plots of cell populations, and expressed as histogram plots in order to quantitate antigen expression. All haematopoietic cells constitutively express specific antigens on their surface, such as glycoproteins or integrins. Some of these are expressed exclusively on particular haematopoietic cells and which may be used to identify a specific cell type. These antigens have been identified as cluster-designated (CD) antigens using monoclonal antibodies.

Platelets exclusively express antigens, such as CD42a and CD61, on their surface in both the activated and non-activated state. Specific antibodies to one of these clusters may be tagged with a fluorochrome which is detected by the flow cytometer. In this way, platelets can be distinguished from the other cells in the blood sample. In addition, recordings of forward scatter indicate the size of the platelets. Single platelets may therefore be identified and recorded. Erythrocytes may also be identified from the forward scatter/side scatter profile and their number recorded. This may then be related to the number of single platelets in the sample. It is possible to therefore monitor platelet aggregation by recording a fixed number of erythrocytes in each blood sample and monitoring the fall in numbers of platelets. The number of single platelets in the sample is reduced as the platelets become incorporated into a platelet aggregate while the number of erythrocytes remains the same.

In many circumstances, particularly in a research laboratory, a time window of two hours would be sufficient to allow for the measurement of platelet aggregation in samples simply preserved by addition of a formaldehyde solution. Aggregation measurements in whole blood could be assessed by a platelet counting method using either an impedance platelet counter or a flow cytometer, and a direct correlation between the two counting methods has been reported. However, there are many circumstances when it would be necessary to have a longer time window, for example, when a study is conducted away from the laboratory in a clinical setting. In some circumstances, such as a clinical trial, this may be in a different country and simple addition of a formaldehyde solution is not sufficient to preserve the cells. Currently, it is not therefore possible to measure platelet aggregation remotely, and so there is a need to stabilise platelets and other blood cells for much longer time periods to enable analysis of blood cell conjugates to be carried out remotely.

When blood cells are removed from the body and exposed to foreign surfaces, it is necessary to add agents to the blood to preserve their structure and function as well as the antigenic makeup of the surface of the cells. Traditionally, anticoagulants were developed to prevent blood from clotting in order to perform, for example, the microscopic examination of blood cells. Most anticoagulants work through inhibition of enzymes involved in the coagulation process. This has been commonly accomplished through the removal or reduction of calcium ions using EDTA or sodium citrate. Other inhibitors of coagulation enzymes have also been used such as sodium fluoride, hirudin or heparin. While these substances act to maintain the blood in a liquid state and prevent clotting, they may also affect other aspects of cell activation such as shape change, granule release and antigen exposure to a varying degree and will not necessarily preserve the level of antigen expression. Anticoagulant use therefore requires careful monitoring and an understanding of the role each type of anticoagulant may play in cell activation and the expression of cell markers. For example, the inhibitory effects of pharmaceutical agents on platelets may be monitored more successfully in some anticoagulants than others.

Various substances may also be added to the blood to preserve pH, which may affect the degree of exposure of antigens and the degree of blood cell conjugations, such as platelet activation, and subsequently aggregation. It is also important to maintain the osmolarity of blood since any changes may result in rupture of blood cell membranes and the ejection of cell contents. This is particularly important in red blood cells which contain large amounts of both ADP and ATP, substances which are known to activate platelets in whole blood. Changes in osmolarity can also lead to changes in cell size through crenation or shrinkage. Buffer solutions are therefore used to preserve pH and osmolarity and these may contain a variety of substances including proteins and salts.

Further substances, such as glutaraldehyde, paraformaldehyde, alcohols or tannins, may be added to blood samples to act as fixatives. These solutions react with proteins within the cells and cross-link the proteins to retain their structure. These changes are irreversible and are similar to the tanning process. While these processes preserve cell structures, they may also cause some denaturing and may even result in a change in the proteins on the surface of the cells. Some antigens may therefore be destroyed by this process while others may remain antigenic, and this will influence the results of flow cytometry analysis. Experience and a degree of understanding of the changes incurred through the use of any fixative is therefore necessary in order to judge its effects on any antigen expression on platelets or other blood cells and on any fluorescent probes used.

Fixative solutions, such as formaldehyde, have been used previously to preserve samples for flow cytometry, and much research has been conducted in an attempt to preserve samples for later analysis. However, no fixative solution has been found that is suitable for long-term storage. Furthermore, in some cases, the fixative has been shown to cause platelet activation itself, which is clearly undesirable. US 6,913,932 discloses a non-lytic platelet stabilisation composition which includes a phosphatase inhibitor, and a protease inhibitor, and reagents that generate formaldehyde-ammonium complexes with the platelets. The authors assert that their stabilisation composition provides stability for at least 24 hours, and that platelets stabilised using this composition may be used for the measurement of platelet CD62P expression. Clearly, there are many situations in which it would be desirable to stabilise platelets and platelet aggregates for much longer than 24 hours in order to provide more time for the transportation of a blood sample from the site at which the sample was taken from a subject to a diagnostic lab where the sample may be analysed by aggregation studies.

US 6,872,572 discloses the use of platelet counting for the assessment of platelet function. Platelets are counted after incubation in the presence or absence of EDTA. However, this document does not disclose adding any platelet aggregate preservative to blood samples. Accordingly, in the absence of an aggregate preservative, any aggregates that have formed would gradually break up. Measurements of single platelet count in this situation following addition of a stimulant would gradually increase as the aggregates break up. Therefore, it would be necessary to count the platelets in the sample immediately. As such, it would not be possible to use the method disclosed in US 6,872,572 to carry out platelet aggregation studies on samples at remote locations where transportation to a remote diagnostic lab is required.

US 2007/0166389 A1 discloses a lyophilized platelet preparation obtained by stabilizing separated platelets with glyceraldehyde.

US 3,925,541 discloses a method of coating double aldehyde stabilized erythrocytes with antigens/and or antibodies.

US 5,503,982 discloses a means of detection of myorcardial infarction by measurements of monocyte/platelet conjugates.

US 2004/0038424 discloses formaldehyde-ammonium salt complexes for the stabilisation of blood cells.

Schmidt V. & Hilberg, T. ThromboFix™ Platelet Stabilizer: Advances in clinical platelet analyses by flow cytometry? Platelets June 2006; 17(4): 266-273 discloses a platelet stabilizer, ThromboFix™ for clinical diagnostics.

It is therefore an object of the present invention to overcome or mitigate one or more of the problems of the prior art, whether identified herein or elsewhere, and to provide improved methods for fixing and stabilising blood cell conjugates (eg platelet aggregates) in a blood sample for extended periods of time. This would enable blood samples to be taken from a subject and transported significant distances, eg to foreign laboratories for blood cell conjugate analysis.

The inventors have devised a new method for fixing and stabilising blood cell conjugates, and in particular for fixing platelet aggregates, for subsequent analysis. The method involves adding two compositions, referred to as a fix solution and a stabiliser solution, sequentially to a blood sample. The number of single platelets in the sample may be subsequently determined using known techniques, such as flow cytometry. The degree of platelet aggregation may then be calculated using the value of single platelets present in the sample. However, as shown in Figures 10 to 15, the method of the invention is not limited to stabilising only platelet aggregates for platelet aggregation analysis, and may be used to stabilise any blood cell conjugate, such as platelet-monocyte conjugates (see Figures 11, 13, and 14), and platelet-neutrophil conjugates (see Figures 12 and 15).

Therefore, according to a first aspect of the invention, there is provided a method of stabilising blood cell conjugates in a sample, wherein blood cell conjugates comprise two or more blood cells joined together wherein the blood cells are one or more of platelets, erythrocytes and leukocytes, the method comprising the steps of:

(i) contacting a sample containing blood cells with a first composition comprising an aliphatic aldehyde and a buffer, to thereby fix blood cell conjugates present in the sample such that any blood cell conjugates present in the sample remain together and cannot fall apart, and any single cells remain unconjugated, either before or during subsequent analysis; and

(ii) contacting the sample fixed in step (i) with a second composition comprising an aliphatic aldehyde, a chelating agent and a buffer, to thereby stabilise the blood cell conjugates wherein the concentration of aliphatic aldehyde in contact with the blood cells after contact with the second composition is less than after step (i).

Advantageously, the method of the first aspect enables the stabilisation of blood cell conjugates, such as platelet aggregates, for significantly longer periods of time (ie over 2 days) than would be possible using prior art methods (ie a maximum or 1-2 days). Accordingly, a blood sample may be taken from a test subject, contacted with the first composition in step (i) of the method followed by the second composition in step (ii) of the method, and stored for long periods of time, prior to subsequent detection of the amount of blood cell conjugates in the sample. Hence, by using the method according to the invention, it is possible to stabilise blood cell conjugates (eg platelet aggregates) for transportation over long distances, if required, before final analysis.

By the term "blood cell conjugate", we mean a collection or mass of two or more blood cells that are joined together.

Blood cells include erythrocytes (red blood cells), leukocytes (white blood cells), and platelets. Hence, the method may be used to stabilise platelet-platelet conjugates (also known as platelet aggregates), platelet-leukocyte conjugates, platelet-erythrocyte conjugates, and/or leukocyte-leukocyte conjugates. The leukocyte in the platelet-leukocyte conjugate or leukocyte-leukocyte conjugate may be a monocyte, a neutrophil or a lymphocyte. Hence, the method may be used to stabilise platelet-monocyte conjugates, platelet-neutrophil conjugates, or platelet-lymphocyte conjugates etc.

Preferably, the method is used to stabilise platelet- platelet conjugates, ie platelet aggregates.

Preferably, the method of the first aspect comprises an initial step of obtaining, from a test subject, the sample containing a plurality of blood cells, which may be erythrocytes, leukocytes and/or platelets. The sample is preferably a blood sample, which may be obtained by venepuncture. Suitably, the sample comprises at least 10, 000 erythrocytes/ml, and even more suitably at least 100, 000 erythrocytes/ml. Preferably, the sample comprises at least 1, 000, 000 erythrocytes/ml, more preferably at least 100, 000, 000 erythrocytes/ml, even more preferably at least 10, 000, 000, 000 erythrocytes/ml, and most preferably at least 3, 000, 000, 000 erythrocytes/ml.

Suitably, the sample comprises at least 1000 platelets/ml, more suitably at least 10, 000 platelets/ml, and even more suitably at least 100, 000 platelets/ml. Preferably, the sample comprises at least 1, 000, 000 platelets/ml, more preferably at least 10, 000, 000 platelets/ml, even more preferably at least 100, 000, 000 platelets/ml, and most preferably at least 200, 000, 000 platelets/ml.

It may be desirable to prevent platelet aggregation from occurring in the blood sample, and so the methods may comprise a step of contacting the sample with an anticoagulant prior to contacting with the first composition in step (i). A suitable anticoagulant may be a solution of sodium citrate or hirudin. Preferably, the blood sample and anticoagulant are mixed to ensure adequate dispersal of the anticoagulant in the blood. The blood sample may be left at room temperature for a fixed time period (eg about 10 minutes, or at least 60 minutes, or any time as appropriate), or processed immediately after venepuncture.

The method of invention may comprise contacting the sample with a suitable blood cell stimulant that is capable of stimulating expression of inducible blood cell surface markers.

By the term "blood cell surface marker", we mean any molecule that is attached to the plasma membrane of a blood cell, and which is characteristic of that blood cell.

By the term "blood cell stimulant", we mean a compound capable of inducing cell signalling pathways such that the blood cell surface markers that are normally internalised or hidden within intracellular stores or granules are released therefrom, and exposed on the blood cell's surface. The blood cell stimulant may also be capable of inducing platelet aggregation.

Examples of suitable blood cell stimulants may be selected from a group of stimulants consisting of calcium ionophore A23187, N- formyl- methionyl- leucyl- phenylalanine (FMLP) , a cytokine (such as TNF- alpha) , ADP, Thrombin Receptor Activating Peptide (TRAP) , U46619, collagen, prostaglandin, epinephrine, Platelet Activating Factor, sodium arachidonate, ristocetin, phorbol myristate acetate (PMA) , 5- hydroxytryptamine (5- HT) , and sulprostone, or any other blood cell stimulant chosen. Blood cell stimulants may be used independently, or in combination, at appropriate concentrations of between about 0.01 $\mu$M and 1 mM.

In many circumstances, the sample is preferably contacted with the blood cell stimulant after the blood cells have been contacted with an anticoagulant. The anticoagulant may be selected from a group of anticoagulants consisting of citrate, hirudin, heparin, Phe-Pro-Arg chloromethyl ketone (PPACK), and sodium fluoride. These anticoagulants would prevent activation of clotting pathways and preserve the cells in a state which would allow them to be stimulated with the blood cell stimulant. EDTA is not a preferred anticoagulant for use at this stage in the method, because in the presence of EDTA conjugation of blood cells is significantly inhibited, even following addition of the blood cell stimulant.

Preferably, an aliquot of the blood sample is added to the blood cell stimulant. The stimulant may be pre- diluted in a volume of solvent (eg saline) , and this volume may be adjusted, if necessary. Alternatively, in some embodiments of the method, a volume of stimulant may be added to the sample instead of the sample to the stimulant. The step of contacting the sample with the blood cell stimulant may be performed at room temperature (ie about 21°C) , or at an elevated temperature (eg above 25°C, above 30°C, or above 35°C) . Preferably, the combined blood and stimulant sample is agitated by shaking and/or stirring for a pre- determined time, eg at least one minute, preferably at least 3 minutes, at least 5 minutes, or at least 10 minutes. Agitation must be sufficient to cause cell- cell contact to occur and therefore allow platelet conjugates to form, but not so aggressive that conjugation is prevented or conjugates broken up once formed. Following contacting with the blood cell stimulant, the methods may then comprise the step of contacting the sample with the first composition in step (i) .

As described in Example 1, the first composition is referred to herein as a "fix solution", and comprises a concentration of aliphatic aldehyde that is sufficiently high to fix any blood cell conjugates (preferably platelet aggregates) present in the blood sample so that they remain fixed together and cannot fall apart either before or during subsequent analysis. Hence, by the expression "to fix a blood cell conjugate", we mean to maintain the blood cell conjugates in their current conjugated form (eg platelet-platelet conjugate, or platelet-leukocyte conjugate etc), and to ensure that any single blood cells (ie unconjugated cells) remain unconjugated. Hence, upon contacting the sample with the first composition in step (i), any blood cell conjugates present within the sample will become fixed. Therefore, blood cell conjugation will be fixed at the level it would be if it had been measured immediately, and so the method may be used to assess the level of blood cell conjugation in samples that have been collected and stabilised for later analysis.

Preferably, the aliphatic aldehyde in the first composition comprises between 1 and 5 carbon atoms. For example, a suitable 5- carbon aldehyde may be glutaraldehyde (ie $C_5H_8O_2$)- Preferably, the aliphatic aldehyde comprises between 1 and 3 carbon atoms, and most preferably one carbon atom, ie formaldehyde (ie $H_2CO$) . Formaldehyde is a gas at room temperature, and it is usually sold as a saturated aqueous solution with a concentration of about 37% formaldehyde, often stabilised with 10- 15% methanol, under the trade name Formalin. Furthermore, it will be appreciated that, in addition to $H_2CO$, formaldehyde may exist as the cyclic trimer trioxane, and the polymeric form, paraformaldehyde.

The concentration of the aliphatic aldehyde in the first composition is preferably at least 0.1 % (v/v), more preferably at least 0.5% (v/v), more preferably at least 1% (v/v). The concentration of the aliphatic aldehyde in the first composition may be between about 0.1 and 15% (v/v), preferably between about 0.5 and 10% (v/v), and most preferably between about 1 and 5% (v/v). Most preferably, a concentration of between 1 % and 3% (v/v) aliphatic aldehyde is used. The inventors have found that such high concentrations of aliphatic aldehyde are suitable for reliably fixing the blood cell conjugates present in the sample.

Preferably, the first composition comprises a solvent. A preferred solvent is water. Hence, the first composition is preferably a buffered solution of the aliphatic aldehyde, which firmly fixes the conjugates in the sample and, in the short term, protects the blood cells from lysis, and also preserves their integrity. Furthermore, the buffer maintains the pH of the composition, and hence blood sample, at physiologically acceptable conditions. Preferably, the pH of the composition is between about 5 and 9, more preferably between about 6 and 8, and most preferably between about 6.5 and 7.5.

The buffer may be selected from a group of buffers consisting of phosphate buffered saline (PBS) , isotonic saline, Tris, N- (2- acetamido)- 2- iminodiaacetic acid, or buffers prepared from pyrophosphate, 4- (2- hydroxyethyl)- 1- piperazineethanesulfonic acid (HEPES) , acetate, imidazole, succinate, maleate, citrate, carbonate, methylethyl sulfide (MES) , 3- (N- morpholino) propanesulfonic acid (MOPS) , or combinations thereof.

The buffer preferably comprises at least one buffering salt, and preferably a plurality of buffering salts, which maintains the composition at physiological pH. Furthermore, the inventors have found that the buffering salts do not adversely affect the blood cell conjugate-fixing function of the first composition. Preferably, the buffer comprises a buffering salt selected from a group of buffering salts consisting of sodium chloride, potassium chloride, disodium hydrogen phosphate, sodium dihydrogen orthophosphate monohydrate, potassium dihydrogen orthophosphate, or combinations thereof. Preferably, the buffer comprises between about 0.1 and 6% (w/v) buffering salt, more preferably between about 0.5 and 4% (w/v), and most preferably between about 1 and 2% (w/v) buffering salt.

Where the buffer comprises sodium chloride, the buffer preferably comprises between about 0.05 and 10% (w/v) sodium chloride, more preferably between about 0.1 and 5% (w/v), and most preferably between about 0.5 and 1% (w/v) sodium chloride.

Where the buffer comprises potassium chloride, the buffer preferably comprises between about 0.001 and 1% (w/v) potassium chloride, more preferably between about 0.01 and 0.5% (w/v), and most preferably between about 0.01 and 0.05% (w/v) potassium chloride.

Where the buffer comprises potassium dihydrogen orthophosphate, the buffer preferably comprises between about 0.001 and 1% (w/v) potassium dihydrogen orthophosphate, more preferably between about 0.01 and 0.5% (w/v), and most preferably between about 0.01 and 0.05% (w/v) potassium dihydrogen orthophosphate.

Where the buffer comprises disodium hydrogen orthophosphate, the buffer preferably comprises between about 0.01 and 2% (w/v) disodium hydrogen orthophosphate, more preferably between about 0.1 and 1% (w/v), and most preferably between about 0.1 and 0.5% (w/v) disodium hydrogen orthophosphate.

Where the buffer comprises sodium dihydrogen orthophosphate monohydrate, the buffer preferably comprises between about 0.01 and 1% (w/v) sodium dihydrogen orthophosphate monohydrate, more preferably between about 0.1 and 0.7% (w/v), and most preferably between about 0.1 and 0.4% (w/v) sodium dihydrogen orthophosphate monohydrate.

The weight ratio of buffer to aliphatic aldehyde in the first composition may be at least 0.1:1, more preferably at least 0.3:1, even more preferably at least 0.5:1, and most preferably at least 0.8:1. The weight ratio of buffer to aliphatic aldehyde may be less than 10:1, more preferably less than 5:1, even more preferably less than 3:1, and most preferably less than 2:1.

Preferably, the first composition comprises 1 part by weight of aliphatic aldehyde, and 0.1- 10 parts by weight buffer. More preferably, the first composition comprises 1 part by weight of aliphatic aldehyde, and 0.5- 3 parts by weight buffer.

Preferably, the first composition comprises at least 80% (v/v) solvent, more preferably at least 90% (v/v) , even more preferably at least 95% (v/v) , and most preferably at least 97% (v/v) solvent.

The weight ratio of solvent to buffer in the first composition may be at least 20:1, more preferably at least 40:1, even more preferably at least 50:1, and most preferably at least 60:1. The weight ratio of solvent to buffer may be less than 140:1, more preferably less than 120:1, even more preferably less than 100:1, and most preferably less than 80:1.

The weight ratio of solvent to aliphatic aldehyde in the first composition may be at least 20:1, more preferably at least 40:1, even more preferably at least 50:1, and most preferably at least 60:1. The weight ratio of solvent to aliphatic aldehyde may be less than 120:1, more preferably less than 100:1, even more preferably less than 80:1, and most preferably less than 70:1.

Hence, one embodiment of the first composition comprises a buffered formalin solution. It will be appreciated that the components of the first composition may be dissolved in any suitable volume of solvent, for example 100ml, and that a suitable volume of the resultant solution may be used in accordance with the invention. A preferred method for preparing one embodiment of the first composition (ie buffered formalin) , and optimum dilutions thereof when used to stabilise platelet aggregates and conjugates, is described in Example 1.

The first composition may be added to the sample of blood cells at a ratio of 1 part blood sample to up to 50 parts first composition, preferably 1 part blood sample to up to 30 parts first composition, more preferably 1 part blood sample to up to 10 parts first composition, and most preferably 1 part blood sample to up to 5 parts first composition. As described in Example 1, the most preferred dilution is 1 part blood to 3 parts first composition.

It is preferred that the first composition remains in contact with the blood sample in step (i) of the method for a period of time that is sufficient for effective fixation of the blood cell conjugates in the sample, but not too long that the high concentration of aliphatic aldehyde in the sample would affect the stability of the sample. In particular, prolonged exposure may cause damage to the red blood cells in the blood. Red blood cells play an important part in calculating the degree of blood cell conjugation, and especially platelet aggregation. Preferably, the sample is in contact with the first composition in step (i) for between about 10s and 60 min, more preferably between about 1 min and 45 min, even more preferably between about 10 min and 40 min, and most preferably between about 15 min and 35 min. Most preferably, the sample is in contact with the first composition for about 30 min.

Following step (i) of the method for fixing the blood cell conjugates, the high concentration of the aliphatic aldehyde (preferably formaldehyde) is diluted away by the addition of the second composition in step (ii) of the method. The second composition may be added to the fixed sample at a ratio of 1 part fixed sample to up to 100 parts second composition, preferably 1 part fixed sample to up to 50 parts second composition, more preferably 1 part fixed sample to up to 30 parts second composition, and most preferably 1 part fixed sample to up to 15 parts second composition. As described in Example 1, the most preferred dilution is 1 part fixed blood to 9 parts second composition. The dilution reduces the concentration of aliphatic aldehyde that is in contact with the blood cells. In embodiments where the method is used for detecting and quantifying platelet aggregation in a blood sample, the inventors have found that it is important to sufficiently dilute the concentration of aliphatic aldehyde in contact with the blood cells so that the red blood cells (erythrocytes) and/or platelets are not damaged and/or destroyed, as these are key to calculating the degree of blood cell conjugation in the sample.

As described in Example 1, the second composition is referred to herein as a "stabiliser solution". The aliphatic aldehyde is provided in the second composition in order to react with blood cell surface markers, and cross-link these proteins thereby stabilising them and retaining their structure. Accordingly, their concentration (or expression level) on the blood cell's surface is maintained for subsequent detection, for example by flow cytometric analysis.

By the term "stabilising expression of a blood cell surface marker", we mean maintaining the level of expression of the blood cell surface marker, and hence concentration of the marker on the blood cell surface. The blood cell surface marker may be expressed (ie exposed on the blood cell surface) constitutively (ie constantly), or its expression may be induced, for example by the presence of a blood cell stimulant.

The aldehyde component of the second composition is also believed to form methylene cross-links with cell proteins, thereby trapping substances, such as carbohydrates and lipids within the forming matrix. Blood cell conjugates, such as platelet aggregates, have been shown to remain stable for at least 9 days following this treatment. Therefore, from the foregoing, it will be appreciated that the second composition provides a stable, non-lytic environment for the blood cells whether conjugated or not, and particularly the platelets and red blood cells, and enables them to be preserved for extended time periods for subsequent analysis. Hence, by the expression "to stabilise a blood cell conjugate", we mean to prevent lysis of the blood cells whether conjugated or not, and to stabilise expression of the blood cell surface markers.

The aliphatic aldehyde in the second composition may be the same as or different to the aliphatic aldehyde in the first composition. Preferably, the aliphatic aldehyde in the second composition comprises between 1 and 5 carbon atoms. For example, a suitable 5-carbon aldehyde may be glutaraldehyde. Preferably, the aliphatic aldehyde comprises between 1 and 3 carbon atoms, and most preferably one carbon atom, ie formaldehyde. The concentration of the aliphatic aldehyde in the second composition may be between about 0.01 and 5% (v/v), preferably between about 0.03 and 1 % (v/v), and most preferably between about 0.05 and 0.5% (v/v). Most preferably, a concentration of between 0.1 and 0.2% (v/v) aliphatic aldehyde is used. The inventors have demonstrated that such concentrations reliably stabilise the blood cell conjugates, and in particular the expression of the cell surface markers on the blood cells.

Preferably the concentration of aliphatic aldehyde in the first composition is higher than the concentration of aliphatic aldehyde in the second composition. Preferably the concentration of aliphatic aldehyde in the first composition is at least double the concentration of aliphatic aldehyde in the second composition, more preferably it is at least 5 times greater, more preferably at least 8 times greater. Preferably the concentration of aliphatic aldehyde in the first composition is about 10 times greater than the concentration of aliphatic aldehyde in the second composition.

The chelating agent in the second composition is capable of forming complexes with metal ions, and therefore inhibits the action of enzymes, such as metalloproteases, which rely on bound metals as cofactors. Hence, the chelating agent acts to preserve the blood cells by preventing degradation processes initiated by enzymatic attack. The chelating agent may also advantageously act as a blood anticoagulant, preventing further blood cell conjugation occurring over time. The chelating agent forms complexes with calcium ions present in blood plasma, and prevents blood coagulation.

The chelating agent may be selected from a group of chelating agents consisting of ethylenediaminetetraacetic acid (EDTA) , ethyleneglycol- bis (ß- aminoethylether)- *N, N, N', N'*- tetraacetic acid (EGTA) , *trans- 1, 2- diamino- cyclohexane- N, N, N'N'- tetraacetic* acid (CDTA) , nitriloacetic acid (NTA) , Porphine, Heme, 1, 2- bis (o- aminophenoxy) ethane- *N, N, N, N*- tetraacetic acid (BAPTA) , Dimercaprol, or combinations thereof. Most preferably, the chelating agent is EDTA.

The concentration of the chelating agent in the second composition may be between about 0.01 and 5% (w/v), preferably between about 0.05 and 3% (w/v), more preferably between about 0.1 and 1% (w/v), and most preferably between about 0.1 and 0.5% (w/v).

Preferably, the second composition comprises a solvent. A preferred solvent is water. Hence, the second composition is preferably a buffered solution of the aliphatic aldehyde and chelating agent, which protects the blood cells from lysis, and preserves their integrity for at least 24 hours. Furthermore, the buffer in the second composition is present in order to maintain the pH of the composition, and hence blood sample, at physiologically acceptable conditions.

The buffer of the second composition may be the same as the buffer of the first composition. The buffer in the second composition may be selected from a group of buffers consisting of phosphate buffered saline (PBS) , isotonic saline, Tris, N- (2- acetamido)- 2- iminodiaacetic acid, or buffers prepared from pyrophosphate, 4- (2- hydroxyethyl)- 1- piperazineethanesulfonic acid (HEPES) , acetate, imidazole, succinate, maleate, citrate, carbonate, methylethyl sulfide (MES) , 3- (N- morpholino) propanesulfonic acid (MOPS) , or combinations of these.

The buffer in the second composition preferably comprises at least one buffering salt, and preferably a plurality of buffering salts, which maintains the composition at physiological pH. Furthermore, the inventors have found that the buffering salts in the second composition do not adversely affect the blood cell surface marker expression stabilising function of second composition. Preferably, the buffer comprises a buffering salt selected from a group of buffering salts consisting of sodium chloride, potassium chloride, disodium hydrogen phosphate, sodium dihydrogen orthophosphate monohydrate, potassium dihydrogen orthophosphate, or combinations thereof. Preferably, the buffer comprises between about 0.05 and 5% (w/v) buffering salt, more preferably between about 0.1 and 3% (w/v), and most preferably between about 0.5 and 1.5% (w/v) buffering salt.

Where the buffer of the second composition comprises sodium chloride, the buffer preferably comprises between about 0.05 and 10% (w/v) sodium chloride, more preferably between about 0.1 and 5% (w/v), and most preferably between about 0.5 and 1% (w/v) sodium chloride.

Where the buffer comprises potassium chloride, the buffer preferably comprises between about 0.001 and 1 % (w/v) potassium chloride, more preferably between about 0.01 and 0.5% (w/v), and most preferably between about 0.01 and 0.05% (w/v) potassium chloride.

Where the buffer comprises potassium dihydrogen orthophosphate, the buffer preferably comprises between about 0.001 and 1% (w/v) potassium dihydrogen orthophosphate, more preferably between about 0.01 and 0.5% (w/v), and most preferably between about 0.01 and 0.05% (w/v) potassium dihydrogen orthophosphate.

Where the buffer comprises disodium hydrogen orthophosphate, the buffer preferably comprises between about 0.001 and 2% (w/v) disodium hydrogen orthophosphate, more preferably between about 0.01 and 1 % (w/v), and most preferably between about 0.05 and 0.2% (w/v) disodium hydrogen orthophosphate.

Where the buffer comprises sodium dihydrogen orthophosphate monohydrate, the buffer preferably comprises between about 0.001 and 1 % (w/v) sodium dihydrogen orthophosphate monohydrate, more preferably between about 0.01 and 0.5% (w/v), and most preferably between about 0.01 and 0.05% (w/v) sodium dihydrogen orthophosphate monohydrate.

The weight ratio of buffer to chelating agent in the second composition may be at least 1:1, more preferably at least 2:1, even more preferably at least 3:1, and most preferably at least 5:1. The weight ratio of buffer to chelating agent may be less than 15:1, more preferably less than 12:1, even more preferably less than 10:1, and most preferably less than 8:1.

The weight ratio of buffer to aliphatic aldehyde in the second composition may be at least 1:1, more preferably at least 3:1, even more preferably at least 5:1, and most preferably at least 6:1. The weight ratio of buffer to aliphatic aldehyde may be less than 12:1, more preferably less than 10:1, even more preferably less than 8:1, and most preferably less than 7:1.

The weight ratio of aliphatic aldehyde to chelating agent in the second composition may be at least 0.1:1, more preferably at least 0.3:1, even more preferably at least 0.5:1, and most preferably at least 0.8:1. The weight ratio of aliphatic aldehyde to chelating agent may be less than 5:1, more preferably less than 3:1, even more preferably less than at least 2:1, and most preferably less than 1:1.

Preferably, the second composition comprises 1 part by weight of aliphatic aldehyde, 0.1- 5 parts by weight chelating agent, and 3- 10 parts by weight buffer. More preferably, the second composition comprises 1 part by weight of aliphatic aldehyde, 0.5- 3 parts by weight chelating agent, and 5- 8 parts by weight buffer.

Preferably, the second composition comprises at least 80% (v/v) solvent, more preferably between at least 90% (v/v) , even more preferably at least 95% (v/v) , and most preferably at least 98% (v/v) solvent.

The weight ratio of solvent to buffer in the second composition may be at least 50:1, more preferably at least 80:1, even more preferably at least 90:1, and most preferably at least 100:1. The weight ratio of solvent to buffer may be less than 250:1, more preferably less than 200:1, even more preferably less than 150:1, and most preferably less than 120:1.

The weight ratio of solvent to chelating agent in the second composition may be at least 300:1, more preferably at least 400:1, even more preferably at least 500:1, and most preferably at least 570:1. The weight ratio of solvent to chelating agent may be less than 800:1, more preferably less than 700:1, even more preferably less than 650:1, and most preferably less than 600:1.

The weight ratio of solvent to aliphatic aldehyde in the second composition may be at least 400:1, more preferably at least 500:1, even more preferably at least 600:1, and most preferably at least 650:1. The weight ratio of solvent to aliphatic aldehyde may be less than 900:1, more preferably less than 800:1, even more preferably less than 750:1, and most preferably less than 700:1.

Hence, one embodiment of the second composition comprises a buffered formalin- EDTA solution. It will be appreciated that the components of the composition may be dissolved in any suitable volume of solvent, for example 100ml, and that a suitable volume of the resultant solution may be used in accordance with the invention, as will be described hereinafter. Example 1 describes a preferred method for preparing one embodiment of the second composition (ie buffered formalin- EDTA solution) , and optimum dilutions thereof when used to stabilise platelet surface markers.

The first and/or second composition used in the method of the first aspect may further comprise an anticoagulant, which may be selected from a group of anticoagulants consisting of citrate, hirudin, heparin, Phe-Pro-Arg chloromethyl ketone (PPACK), and sodium fluoride. Information about anticoagulants is commonly available, and different anticoagulants may be used at different concentrations in the compositions used in the method of the first aspect. By way of example, where the anticoagulant is sodium citrate, it may be provided at a concentration in the blood of about 0.3% (w/v), whereas if the anticoagulant is hirudin, it may be provided at a concentration of about 50μg/ml.

The first and/or second composition may comprise an antioxidant. Examples of suitable antioxidants include ascorbic acid and butylated hydroxytoluene. Where the first or second composition comprises an antioxidant, the composition preferably comprises between about 0.001 and 2 % (w/v) antioxidant, more preferably between about 0.01 and 1 % (w/v), and most preferably between about 0.05 and 0.2 % (w/v) antioxidant.

The first and/or second composition may comprise a preservative, which may be selected from a group of preservatives consisting of sodium azide, sodium benzoate and methyl hydroxybenzoate. Where the first or second composition comprises a preservative, the composition preferably comprises between about 0.001 and 2 % (w/v) preservative, more preferably between about 0.01 and 1% (w/v), and most preferably between about 0.05 and 0.2 % (w/v) preservative.

Samples that have been stabilised with the second composition in step (ii) may be stored at a temperature of between about 2°C and 40 °C (preferably between about 4°C and 25°C), until subsequent analysis is carried out. Samples may be transported in a cool box or on ice to a central laboratory for analysis to take place. The sample may be capable of being stored for at least 1, 2, 3, 4, or 5 days. Preferably, the sample may be capable of being stored for at least 6, 7, 8, 9, or 10 days.

In some embodiments of the method, the degree of sample dilution or the ratio of diluted cells to stabilisation composition may need to be varied. The type and amount of blood cell stimulant added to the sample prior to the stabilisation composition may also be varied according to the requirements of the analysis. Additionally, the method may comprise a step of contacting the sample with substances other than a blood cell stimulant. For example, substances, such as known blood cell inhibitors or blood cell enhancers of blood cell activation, may be added to provide further information on blood cell conjugate formation (including platelet aggregation). A blood cell enhancer is a compound which, on its own, will not stimulate a blood cell, but in combination with a stimulant, will enhance the degree of stimulation. A blood cell inhibitor is a compound, which in combination with a blood cell stimulant, will inhibit the degree of stimulation. In some embodiments, the sample may be collected from a subject and contacted with the first and second compositions for the evaluation of blood cell aggregation in the absence of stimulation by a blood cell stimulant.

Preferably, the method comprises a step of detecting the amount of blood cell conjugates stabilised in the sample resulting from step (ii) of the method. Preferably, said detection step is carried out using flow cytometry. The method may therefore comprise contacting the sample with a blood cell identifier molecule that is capable of identifying blood cells and/or a specific blood cell surface marker present on the blood cells. Preferably, the cell identifier molecule is added to the stabilised sample after step (ii) . Preferably, the blood cell identifier molecule is an antibody or a functional fragment thereof.

The choice of blood cell identifier molecule will depend on which blood cell conjugates are to be detected, eg platelet- platelet, platelet- leukocyte, or platelet- erythrocyte conjugates, etc. For example, an example of an erythrocyte cell surface marker which may be used for identification purposes is CD235a. Hence, the cell identifier molecule may be specific for CD235a in embodiments where the detection step is for detecting blood cell conjugates comprising erythrocytes (eg platelet- erythrocyte conjugates) . Examples of leukocyte markers include CD45 (all leukocytes) , CD14 (specific for monocytes) , CD15 (for neutrophils) , and CD4 or CD8 (for lymphocytes) . Combinations of these markers together with measurement of forward and side scatter profiles for different cell types provide flow cytometry plots suitable for identifying specific cell types, as described in the Examples. Examples of platelet cell surface markers that are expressed constitutively (ie both in the activated and non- activated state) are CD61, CD41 or CD42a. Examples of platelet cell surface activation markers include CD62P (P- selectin) , CD63, or PAC- 1. Hence, the blood cell identifier molecule may be specific for any of CD61, CD41, CD42a, CD62P (P- selectin) , CD63, or PAC- 1,  when detecting platelet- containing conjugates, such as platelet aggregates, erythrocyte- platelet conjugates, monocyte- platelet conjugates, neutrophil- platelet conjugates or lymphocyte- platelet conjugates.

The blood cell identifier molecule is preferably labelled with a detectable label, such as a fluorochrome. Suitable fluorochromes which may be used include Peridinin-chlorophyll protein complex (PerCP), Fluorescein isothiocyanate (FITC), phycoerythrin (PE), and the tandem dye allophycocyanin-cyanine 7 (APC-Cy7), or any other fluorochromes found to fluoresce satisfactorily for flow cytometric detection.

It is preferred that the method of the invention is used to stabilise and detect platelet aggregates. Hence, a preferred platelet identifier molecule which may be used is CD61-PerCP or CD61-FITC, or CD42a-PerCP or CD42a-FITC. However, where the method is used to detect erythrocyte-containing conjugates, a preferred erythrocyte identifier molecule may be CD235a-FITC. Where the method is used to detect monocyte-containing conjugates, a preferred monocyte identifier molecule may be CD14-APCCy7, and so on.

Blood cell identifier molecules (eg antibodies) are added to flow cytometry tubes. In some circumstances, a control blood cell identifier molecule, such as an appropriate immunoglobulin (IgG) labelled with the same fluorochrome, may be used to check for non-specific binding of the antibody and to set a baseline for blood cell conjugation. The stabilised cell sample may be mixed ensuring that any sedimented cells are re-suspended, and the sample may then be added to the flow cytometry tubes containing the antibodies and incubated (eg for 20-30 minutes at 4°C, in the dark). Following incubation, a suitable flow cytometry diluent (for example, that which is sold under the trade name, FACSflow) may be added to the tubes immediately prior to reading on a flow cytometer. Preferably, the flow cytometer is equipped with a blue/green laser operating at an excitation wavelength of 488nm, and has detectors for measurement at suitable wavelengths for the selected fluorochromes. In some circumstances, it may also be necessary to have a second red laser, for example at an excitation wavelength of 633nm, for antibodies conjugated to fluorochromes, such as APC-Cy7. Appropriate software may be used to analyse the data from dot plots. Data acquisition may be stopped after recording 50,000 erythrocyte events (each cell that passes the laser is recorded as an "event"). Example 3 describes how to determine the proportion of conjugated blood cells in the blood sample based on the flow cytometry data.

Where the method is used to detect platelet aggregation, a blood sample resulting from step (ii) may be dispensed into flow cytometry tubes and centrifuged (eg for 10 min at 380g). A pre-determined volume of supernatant may be removed, taking care not to disturb the red blood cell pellet. The blood cell pellet is then incubated with appropriate antibodies for platelet identification. The detection step may comprise determining the number of single platelets present in the sample, and then determining the extent of platelet aggregation based on the number of single platelets, as described in Example 3. The single platelet count may be detected using flow cytometry, and this is a record of the number of single platelets present in the blood sample (recorded as single platelet events, ie the number of platelets passing the laser) in relation to 50,000 erythrocyte events (ie the number of erythrocytes passing the laser) recorded. It may be preferred to calculate a true platelet count by using standard flow cytometry counting beads, such as those sold under the trade name Trucount. Aggregation is usually determined and expressed as a percentage, and in whole blood this may be recorded as a percentage fall in single platelets using the formula shown in Example 3.

As described herein, the method of the invention is not limited to detecting only platelet-platelet conjugates (ie platelet aggregation), and it may also be used to detect other blood cell conjugates. Platelet-leukocyte conjugates may be measured using different flow cytometric procedures to those employed to measure platelet aggregation. These procedures involve the use of leukocyte-identifying markers, such as CD14, which is specifically expressed on monocytes, or other markers, such as the leukocyte marker CD45, may be used to identify the different types of leukocyte present in the blood samples. In addition, specific platelet markers such as CD42a, CD61 or CD41 may be used to identify the presence of platelets, eg platelet-leukocyte conjugates. When both types of markers are co-expressed on one event, then this would indicate that a conjugate of both platelets and leukocytes is present. Blood samples fixed and stabilised by the method of the invention may be incubated with appropriate antibodies and then analysed using an appropriate flow cytometer.

Hence, where the method is used to detect leukocyte- containing blood cell conjugates, a blood sample may be dispensed into a flow cytometry tube and centrifuged (eg for 10min at 380g) . A pre- determined volume of supernatant may be removed, taking care not to disturb the red blood cell pellet. Suitably labelled blood cell identifier molecules, such as CD14- APCCy7 (used to distinguish monocytes and neutrophils) and CD42a- FITC or CD62P- PE (used to identify platelets bound to either monocytes or neutrophils) , may then be added to the tubes and incubated with the cells. As described in Example 11, antibodies to CD14, CD62P and CD42a conjugated to APC- Cy7, PE and FITC, respectively may be used. However, it should be appreciated that other platelet- specific antibodies may also be used (eg CD61) , and fluorochromes other than APC- Cy7, PE and FITC may be substituted provided that they are found to be suitable for the analysis of fixed and stabilised samples. Appropriate assessment by an experienced flow cytometry expert of the suitability of any other platelet or leukocyte- specific antibody or any other fluorescent probe would therefore be necessary before their use in the analysis of these fixed and stabilised samples.

When determining platelet-leukocyte conjugates, it is known that erythrocytes are of a similar size to the conjugates and can make identification of such conjugates on dot plots of forward scatter/side scatter difficult. Accordingly, preferably the method comprises a step of removing or lysing any erythrocytes that may be present in the sample prior to the detection step. Removing the erythrocytes significantly simplifies the flow cytometric analysis of the platelet-leukocyte conjugates. Preferably, the stabilised blood sample is contacted with an agent capable of lysing erythrocytes. For example, following incubation with antibodies, a solution sold under the trade name Erythrolyse solution (available from AbD Serotec, Oxford, UK) may be added to the cells and left to stand (for 10 minutes) to lyse any red blood cells. However, any other suitable erythrocyte lysing agent may be used, such as that sold under the trade name FACS-lysing solution (available from Becton Dickinson, USA). A flow cytometry diluent such as that sold under the trade name FACSflow, or some other suitable flow cytometry diluent may then be added, and the cells may be centrifuged. Supernatants may be removed and cells may be re-suspended in FACSflow to wash the cells. The cells may then be centrifuged again, the supernatant may be removed, and the cells may be finally re-suspended in FACSflow to await reading on the flow cytometer.

Another approach would be to avoid lysing and further centrifugation procedures, and use a leukocyte-identifying antibody, such as CD45, to distinguish all of the leukocytes and platelet-leukocyte conjugates, and use a CD45-triggering technique. This method involves incubating the cells with a fluorochrome-tagged antibody to the universal leukocyte marker CD45 in addition to the other antibodies required for identification of monocytes, neutrophils and platelets. FACSflow may then be added to dilute the cells, which may then be applied to the flow cytometer. Only CD45 positive events are collected using the flow cytometry software, thus eliminating the erythrocytes. The platelet-leukocyte conjugates are then identified from the CD45 positive events using the appropriate leukocyte and platelet antibodies.

Any suitable antibody may be used to identify leukocyte conjugates. There are many examples of leukocyte surface markers that are expressed constitutively, such as CD14 on monocytes, CD15 on neutrophils, and lymphocyte markers, such as CD4 and CD8. Conjugates with other cells, for example platelet-erythrocyte conjugates could be analysed using appropriate erythrocyte antibodies, such as CD235a. All of these markers are described in reference material on cluster differentiation (CD) markers and human leukocyte differentiation antigens, and will be known to the skilled technician, for example on the website: www.hlda8.org/, or in articles such as Zola H et al "CD molecules 2005: human cell differentiation molecules", Blood (2005), 106:3123-3126.

The inventors have devised a kit which is for use in the method of the first aspect, and which may be used to stabilise blood cell conjugates, for blood cell conjugate analyses.

Hence, according to a second aspect of the invention, there is provided a blood cell conjugate stabilisation kit, the kit comprising a first container in which a first composition comprising an aliphatic aldehyde and a buffer is contained, and a second container in which a second composition comprising an aliphatic aldehyde, a chelating agent and a buffer is contained, and optionally instructions for use; wherein the concentration of aliphatic aldehyde in the first composition is higher than the concentration if aliphatic aldehyde in the second composition.

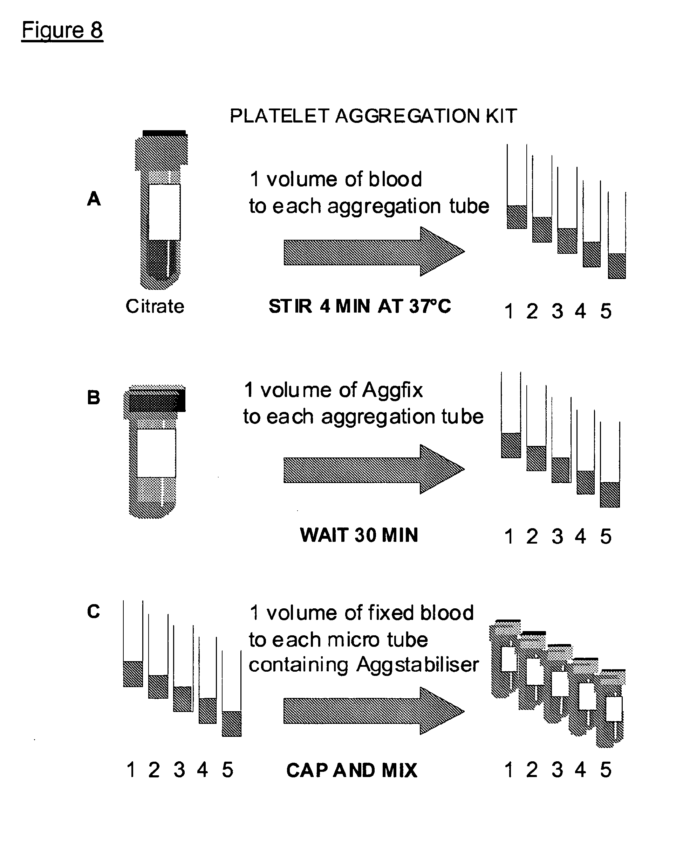

Preferably, the kit of the second aspect is a platelet aggregate stabilisation kit, one embodiment of which is described in Example 10 and illustrated in Figure 8. The inventors believe that this kit is an important aspect of the invention.

Hence, in a third aspect, there is provided a platelet aggregate stabilisation kit, the kit comprising a first container in which a first composition comprising an aliphatic aldehyde and a buffer is contained, and a second container in which a second composition comprising an aliphatic aldehyde, a chelating agent and a buffer is contained, and optionally instructions for use.

Many anti-thrombotic therapies are currently designed to reduce the degree of platelet activation and aggregation, in order to inhibit the formation of a thrombus. The inventors believe that the kit of the second or third aspect may be used to monitor the degree of platelet aggregation following anti-thrombotic therapy. As described in Example 6, the inventors have shown that the method of the first aspect and kit of the second or third aspect may be used to confirm that administration of anti-thrombotic therapy, such as administration of aspirin to a patient, has been sufficient, prior to cardiac intervention procedures, such as angioplasty or stent insertion. Alternatively, the kit may be used to confirm that prescribed anti-thrombotic therapy has been satisfactory following myocardial infarction or stroke. Hence, preferably the kit of the second or third aspect may be used in anti-platelet aggregation drug testing.

The kit of the second or third aspect may comprise blood sample extraction means for obtaining a blood sample from a test subject. The sample extraction means may comprise a needle or syringe or the like. The kit preferably comprises a sample collection container for receiving the extracted blood sample. The sample collection container may contain an anticoagulant in order to prevent the blood sample from clotting. An example of a suitable anticoagulant includes sodium citrate. Preferably, the kit comprises at least one cell activation container containing a blood cell stimulant, and preferably a plurality of activation containers, each containing a different blood cell stimulant. Examples of suitable blood cell stimulants may be selected from a group of blood cell stimulants consisting of ADP, TRAP, U46619, collagen, prostaglandin, epinephrine, Platelet Activating Factor, sodium arachidonate, ristocetin, PMA, 5-HT, and sulprostone or any other suitable stimulant. Blood cell stimulants may be used independently or in combination, at appropriate concentrations of between 0.01 $\mu$M and 1 mM.

Preferably, the kit comprises a positive control container containing a stimulant that is known to induce aggregation or conjugate formation of the cells in the sample. The kit may also comprise a control container containing a compound which prevents platelet aggregation (eg EDTA) which is used to obtain an estimate of the total number of single platelets in an unstimulated sample. Preferably, the kit comprises a negative control container containing a compound which does not induce aggregation or conjugate formation, such as saline. The kit may also comprise a further control container in which a combination of the blood cell stimulant compound and an anti- thrombotic compound (eg aspirin) may be contained.

In use, an operator may obtain a blood sample from the subject using the extraction means. The extracted cell sample may then be introduced into the collection container. The operator may contact the blood sample with anticoagulant. The operator may then contact the blood cell sample with a suitable blood cell stimulant under conditions to stimulate the blood cells. The stimulant may be transferred to the sample collection container, or the sample may be transferred to one or more cell activation containers. Suitable conditions for stimulation may include a temperature of between 21°C and 40°C. Once the blood cell sample has been stimulated, it is then contacted with the first composition to fix the blood cell conjugates, preferably platelet aggregates. Once fixed, the sample is contacted with the second composition to stabilise the blood cell conjugates. Once stabilised, the collection tube containing the cell stimulant and the fixed, stabilised sample may then be stored, and transported to a laboratory equipped to determine the level of aggregation.

It will be appreciated that the inventors have devised a method for stabilising any blood cell conjugates (eg erythrocyte-, leukocyte- or platelet-containing conjugates), for subsequent analysis. However, it is most preferred that the method is used for fixing and stabilising platelet aggregates.

Hence, in a fourth aspect, there is provided a method of stabilising platelet aggregates in a sample, the method comprising the steps of:

(i) contacting a sample containing platelets with a first composition comprising an aliphatic aldehyde and a buffer, to thereby fix platelet aggregates present in the sample; and
(ii) contacting the sample fixed in step (i) with a second composition comprising an aliphatic aldehyde, a chelating agent and a buffer, to thereby stabilise the platelet aggregates.

Preferably, following step (ii) , the method comprises a detection step (iii) for detecting the level of platelet aggregation in the sample.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the following Examples and accompanying Figures.

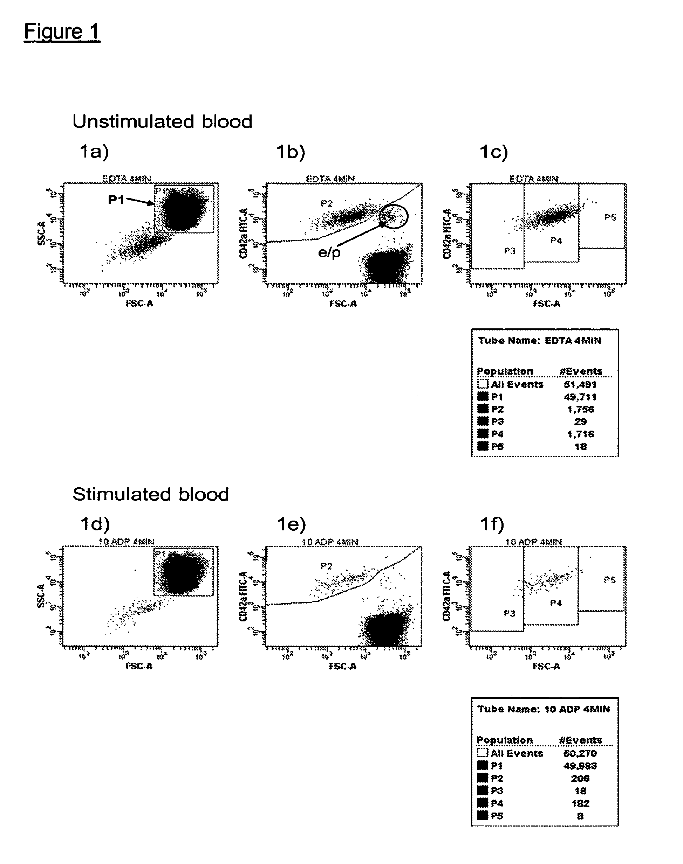

Figure 1 represents the quantification of single platelets in whole blood by flow cytometry. Figure 1 a is a dot plot of forward scatter (FSC) plotted against side scatter (SSC) showing 50,000 red cell events collected in region P1. Figure 1b is a dot plot of FSC plotted against CD42a fluorescence of the platelet marker CD42a. Figure 1c is a dot plot of FSC plotted against CD42a-positive platelet events. Figures 1d, 1e and 1f show an example of a blood sample that has been stimulated with the stimulant ADP, and which had been fixed using the method according to the invention.

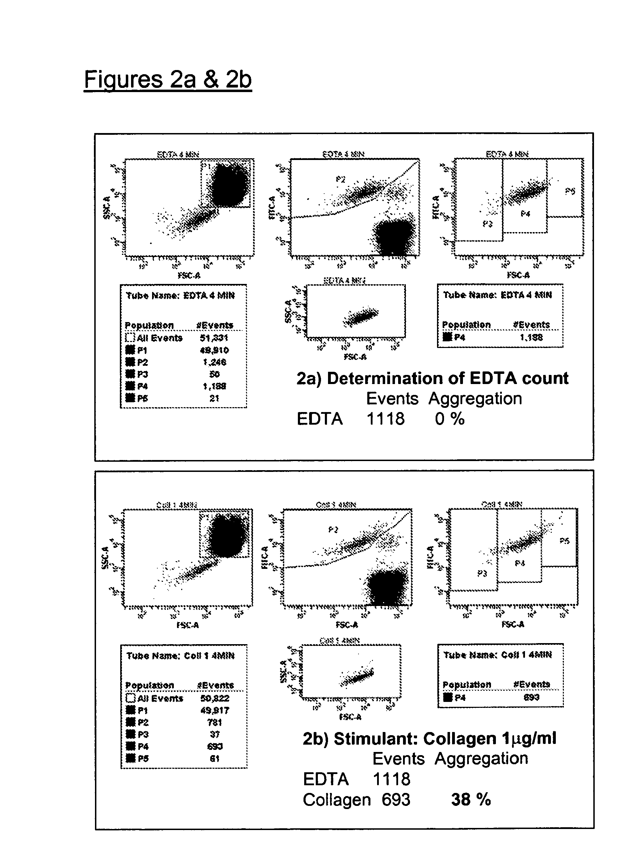

Figures 2a & 2b show examples of dot plots used to determine the number of platelet events for a sample of citrate-anti-coagulated blood containing EDTA to provide a starting count (Figure 2a), which was fixed and stabilised using the method of the invention, and after stimulation of a sample of citrate-anticoagulated blood with 1$\mu$g/ml of the platelet stimulant collagen (Figure 2b).

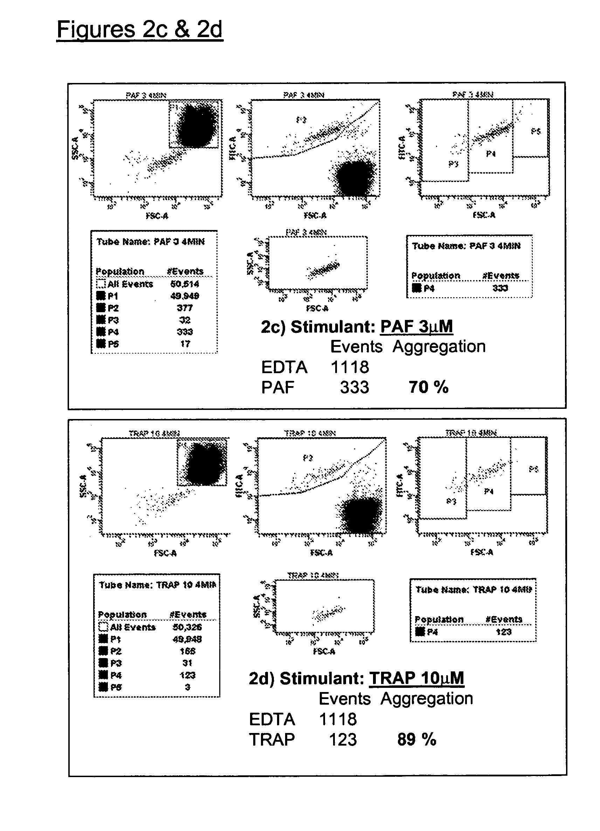

Figures 2c & 2d show further examples of dot plots used to determine the number of platelet events and percentage platelet aggregation for fixed and stabilised citrate-anticoagulated blood samples from the same donor, but stimulated with either 3$\mu$M PAF (Figure 2c) or 10$\mu$M TRAP (Figure 2d).

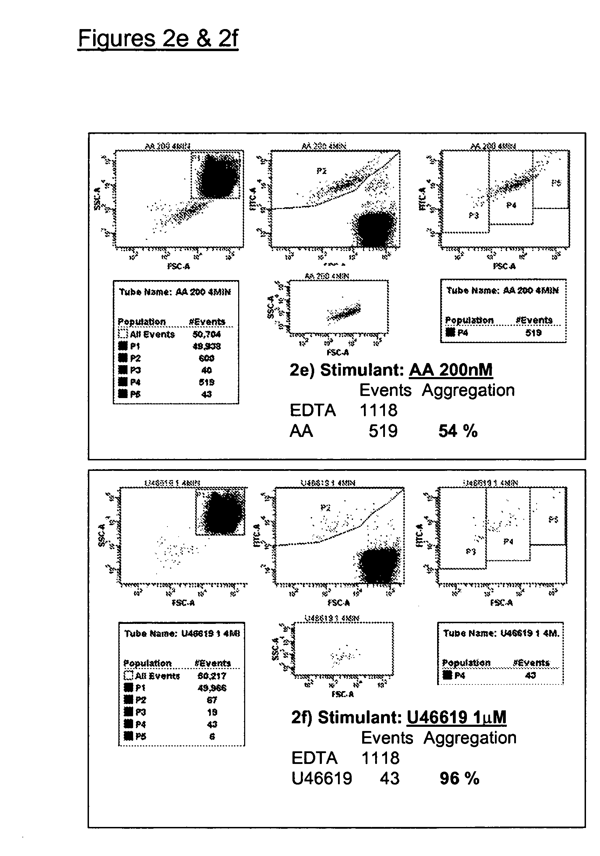

Figure 2e & 2f show further examples of dot plots used to determine the number of platelet events and percentage platelet aggregation for fixed and stabilised citrate-anticoagulated blood samples from the same donor, but stimulated with either 200nM arachidonic acid (AA, Figure 2e) or 1$\mu$M U46619 (Figure 2f).

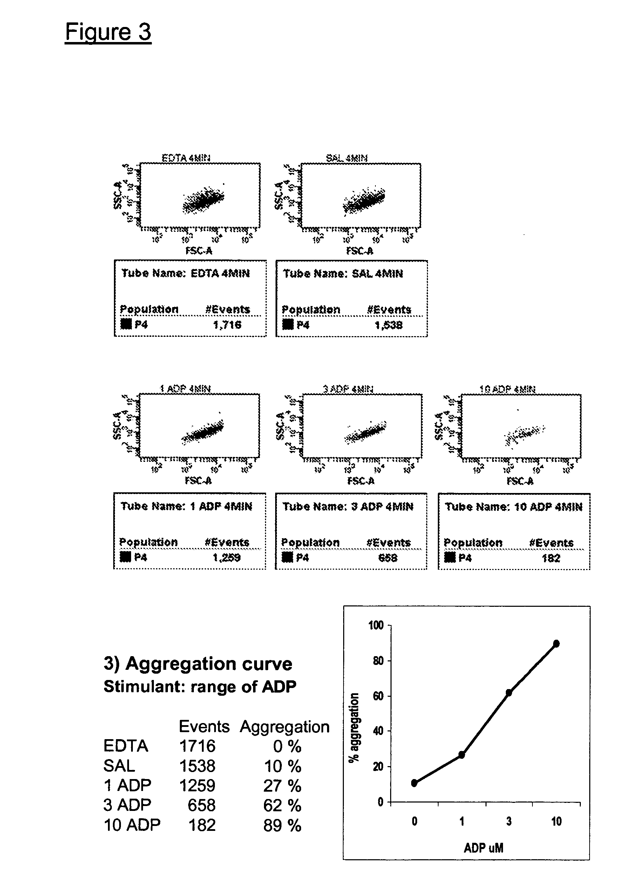

Figure 3 shows examples of dot plots used for determining the number of single platelets in whole blood by flow cytometry, in which citrate-anti-coagulated blood samples had been fixed and stabilised using the method of the invention. Samples of citrate anti-coagulated blood were also stirred for 4 minutes with saline (0) and 1, 3 and 10$\mu$M ADP, and then fixed and stabilised.

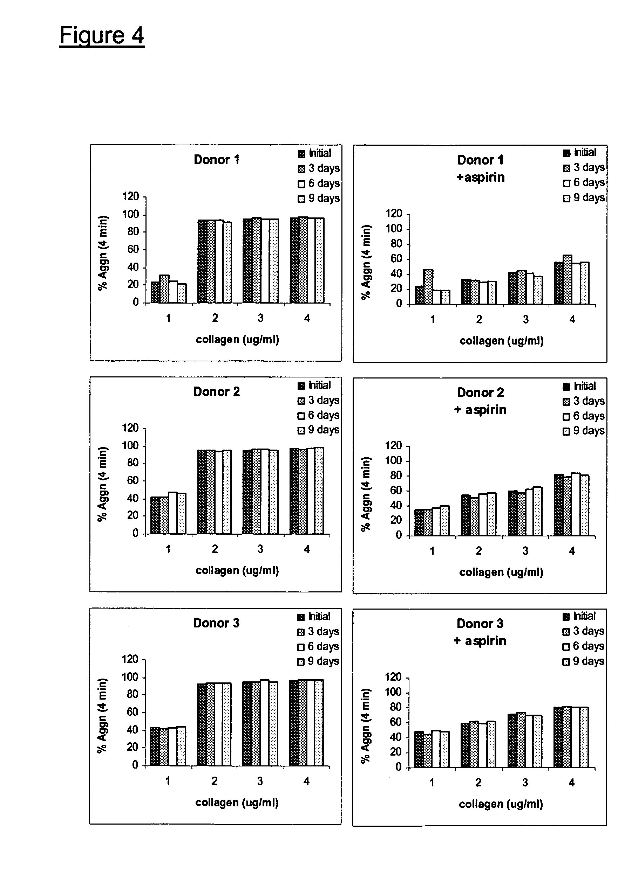

Figure 4 shows bar charts showing the stability of aggregation measurements over 9 days in citrate-anti-coagulated blood following stimulation with a range of collagen concentrations for 10 minutes in the absence (left) or presence (right) of aspirin. Results were obtained from three different donors, Donor 1 to Donor 3.

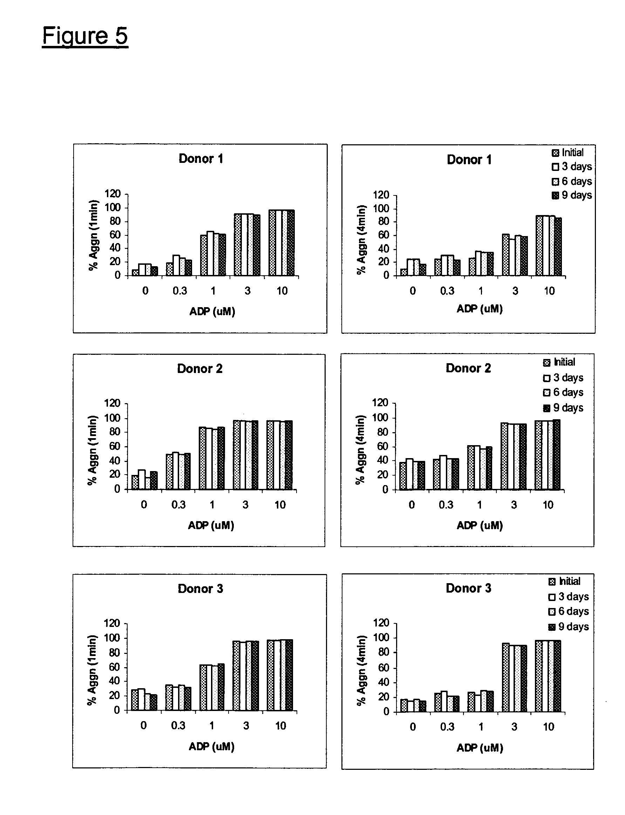

Figure 5 shows bar charts showing the stability of aggregation measurements over 9 days in citrate-anti-coagulated blood, following stimulation with a range of ADP concentrations (0.3-10$\mu$M) for 1 minute (left) and 4 minutes (right). Results were obtained from three different donors.

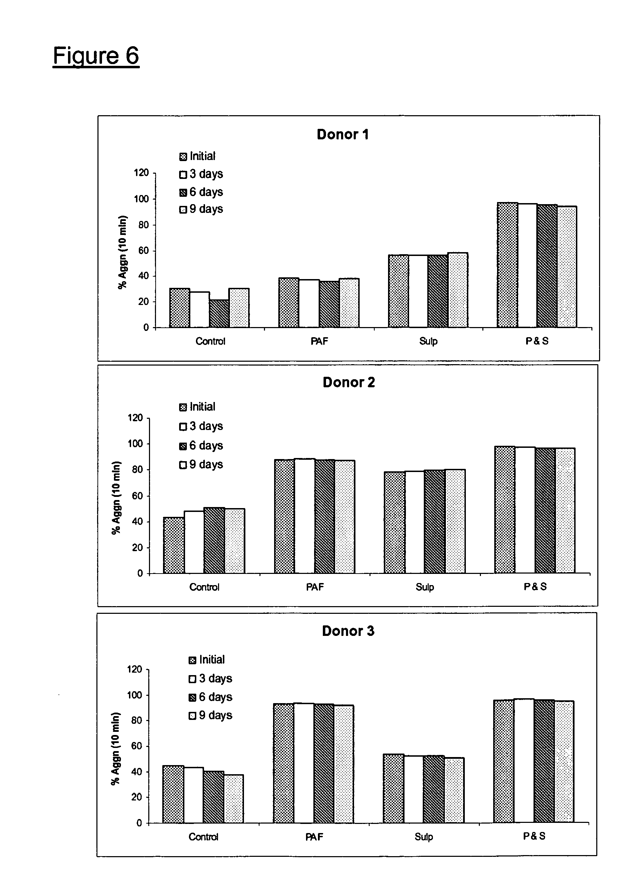

Figure 6 shows bar charts showing the stability of aggregation measurements over 9 days in citrate-anti-coagulated blood following stimulation with saline (control), Platelet Activating Factor (PAF), Sulprostone (Sulp), or a combination of both stimulants, for 10 minutes. Results were obtained from three different donors.

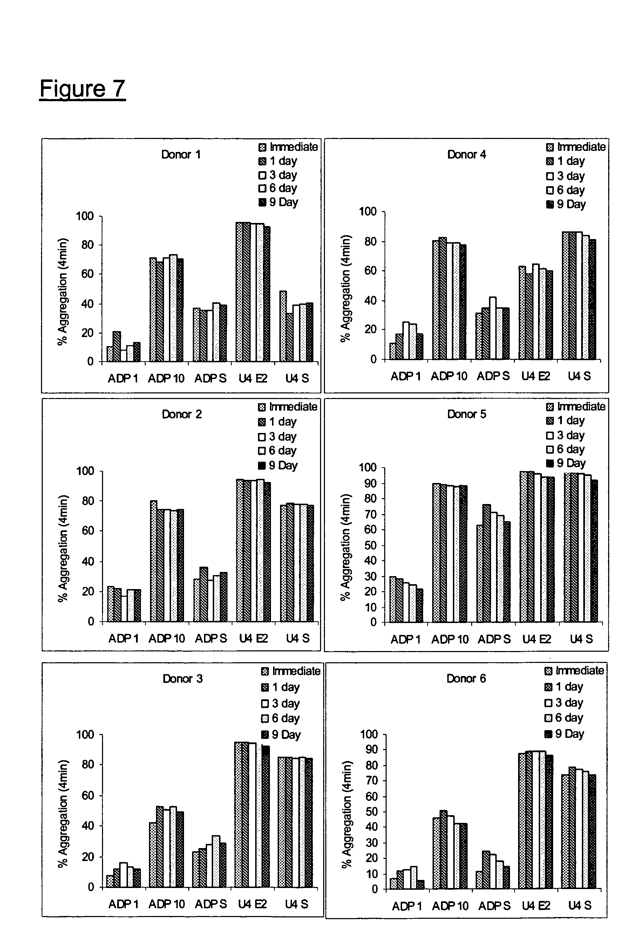

Figure 7 shows bar charts showing the stability of aggregation measurements over 9 days in citrate-anti-coagulated blood following stimulation with ADP1, ADP 10, a combination of ADP and Sulprostone (ADP S), a combination of U46619 (U4) and prostaglandin E2 (U4 E2), or U46619 and Sulprostone (U4 S), for 4 minutes. Results were obtained from six different donors.

Figure 8 shows an example of a protocol sheet for a platelet aggregation kit.

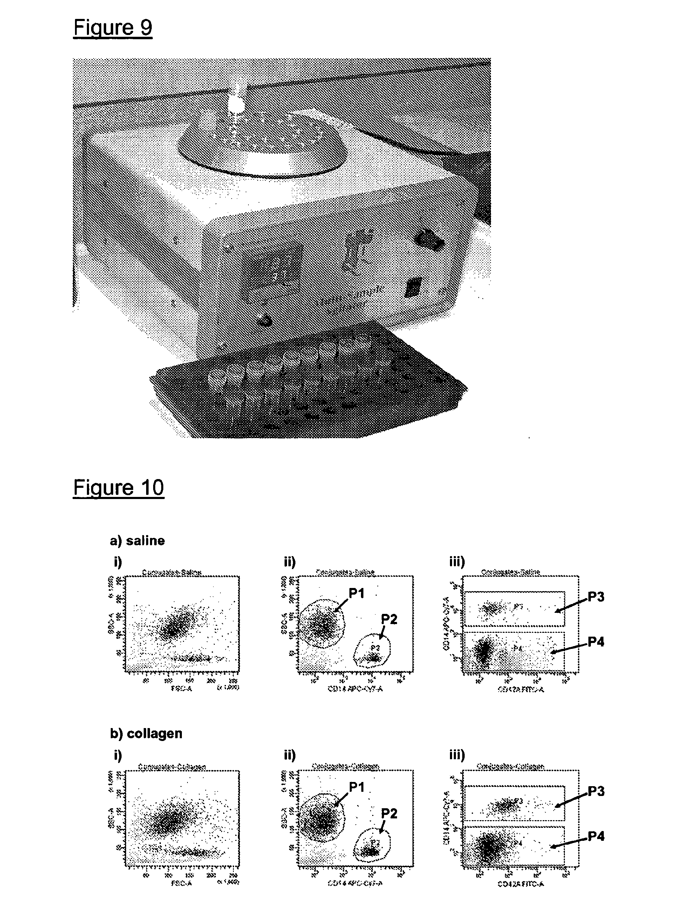

Figure 9 shows a multisample agitator (MSA), for carrying out platelet aggregation studies in accordance with the invention.

Figure 10 shows dot plots used for identifying platelet-leukocyte conjugates in fixed and stabilised blood samples following incubation with (a) saline and (b) collagen. Dot plots of (i) forward-scatter versus side scatter, (ii) CD14-APCCy7 versus side scatter, and (iii) CD42a-FITC versus CD14-APCCy7 are shown.

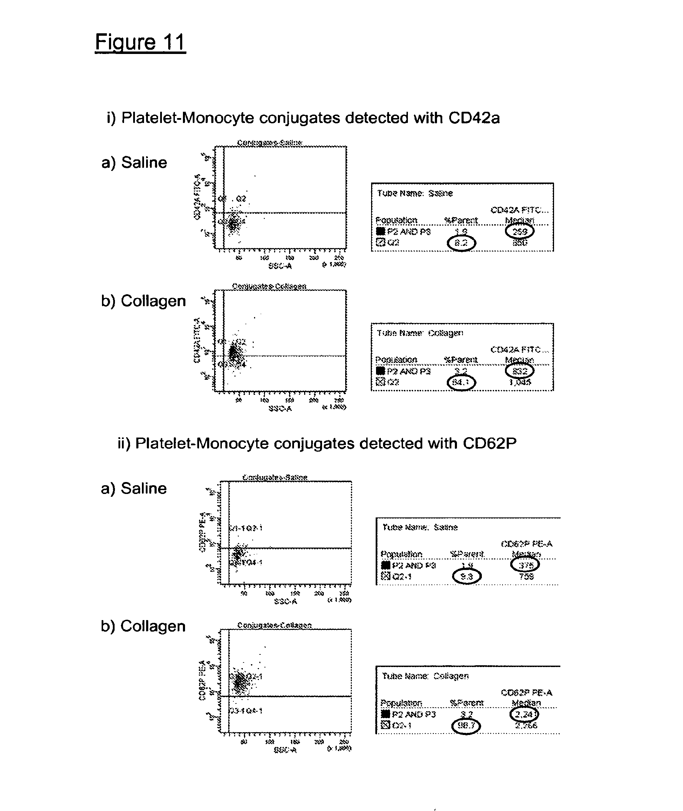

Figure 11 shows the quantification of platelet-monocyte conjugates (identified from gates P2 and P3 as shown in Figure 10 which are detected with the platelet-specific markers CD42a or CD62P. Figure 11 (i) shows CD42a quantification of platelet-monocyte conjugates after incubation with (a) saline or (b) collagen. These are shown as the percentage of the parent population of monocytes captured in gate Q2 (% gated), eg (a) 8.2, (b) 64.1, or as the median fluorescence (mf) of the monocyte population P2 and P3 median, eg (a) 259, (b) 832 circled in the statistics boxes. Figure 11(ii) shows CD62P quantification of platelet-monocyte conjugates after incubation with (a) saline or (b) collagen. These are shown as the percentage of the parent population of monocytes captured in gate Q2-1 (% gated),eg (a) 9.3, (b) 98.7, or as the median fluorescence (mf) of the monocyte population P2 and P3 median, eg (a) 375, (b) 2,241, circled in the statistics boxes.

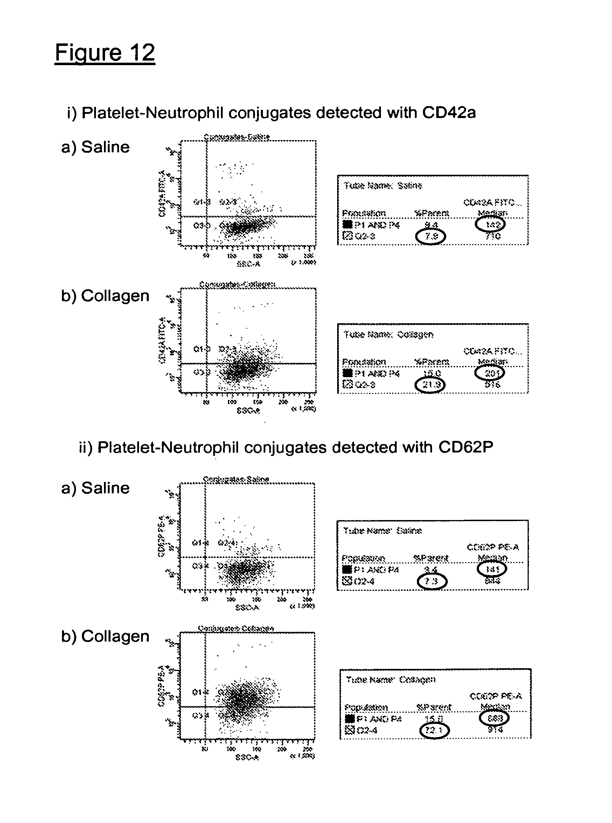

Figure 12 shows the quantification of platelet-neutrophil conjugates. Figure 12(i) shows CD42a quantification of platelet-neutrophil conjugates after incubation with (a) saline or (b) collagen. These are shown as the percentage of the parent population of neutrophils captured in gate Q2-3 (% gated), eg (a) 7.9, (b) 21.9, or as the median fluorescence (mf) of the neutrophil population P1 and P4 median, eg (a) 142, (b) 201, circled in the statistics boxes. Figure 12(ii) shows CD62P quantification of platelet-neutrophil conjugates after incubation with (a) saline or (b) collagen. These are shown as the percentage of the parent population of neutrophils captured in gate Q2-4 (% gated), eg (a) 7.3, (b) 72.1, or as the median fluorescence (mf) of the neutrophil population P2 and P3 median, eg (a) 141, (b) 688, circled in the statistics boxes

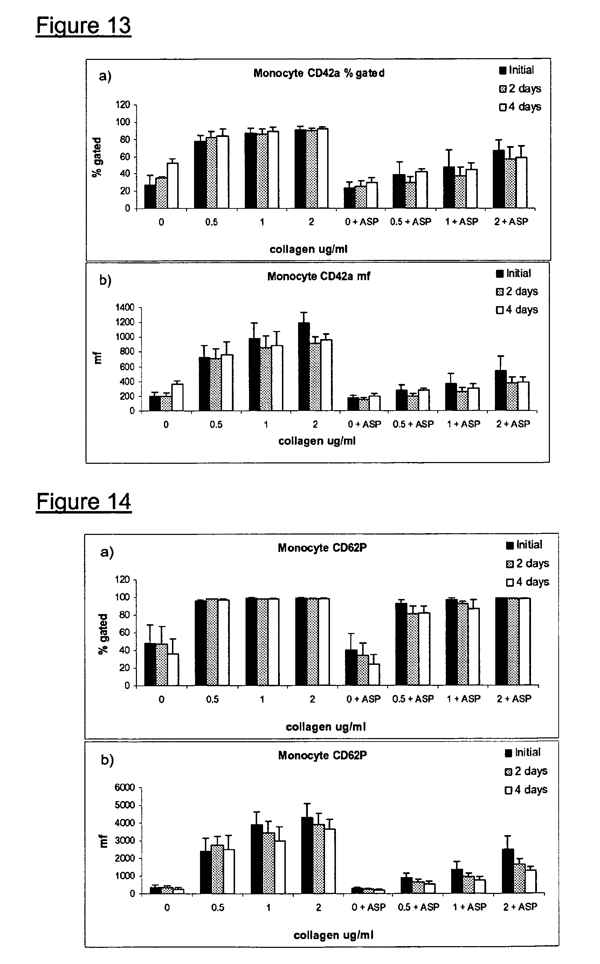

Figure 13 shows bar charts showing the stability of platelet-monocyte conjugates induced by stirring citrate-anticoagulated blood with collagen or collagen plus aspirin. These were measured within 5 minutes of fixing and stabilising the stirred blood, and after storage at 4°C for 2 days and 4 days.

Figure 13(a) shows the percentage of monocytes conjugated with platelets measured as a percentage of monocytes expressing CD42a (% gated), and

Figure 13(b) shows the percentage of monocytes conjugated with platelets using CD42a median fluorescence (mf).

Figure 14 shows bar charts showing the stability of platelet-monocyte conjugates induced by stirring citrate-anticoagulated blood with collagen or collagen plus aspirin. These were measured within 5 minutes of fixing and stabilising the stirred blood, and after storage at 4°C for 2 days and 4 days.

Figure 14(a) shows the percentage of monocytes conjugated with platelets measured as a percentage of monocytes expressing CD62P (% gated), and

Figure 14(b) shows the percentage of monocytes conjugated with platelets using CD62P median fluorescence (mf).

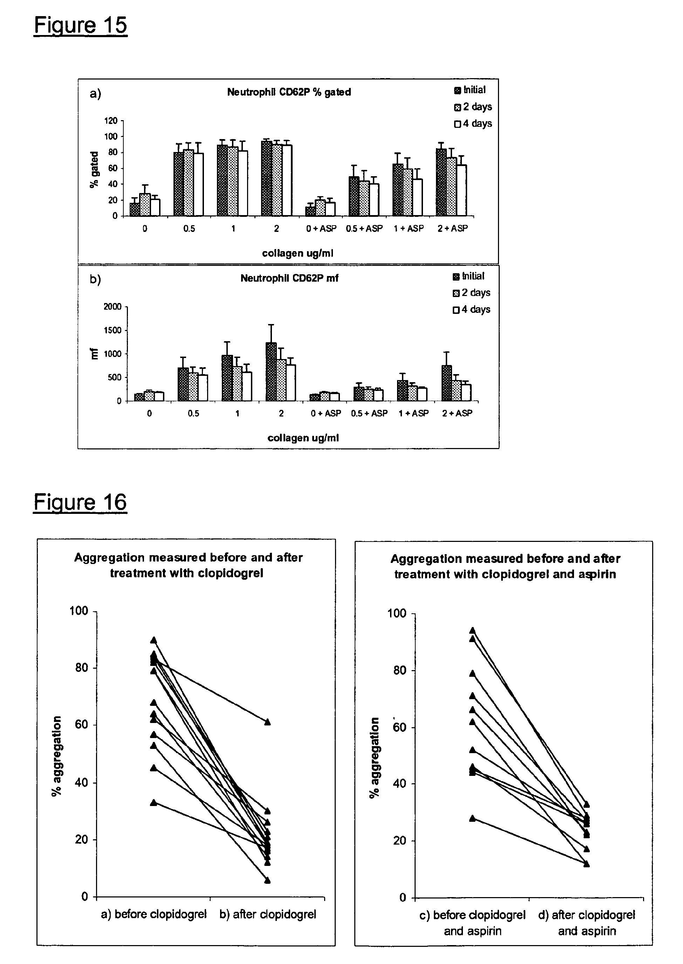

Figure 15 shows bar charts showing the stability of platelet-neutrophil conjugate formation induced by stirring citrate-anticoagulated blood with collagen or collagen plus aspirin. These were measured within 5 minutes of fixing and stabilising the stirred blood, and then after storage at 4°C for 2 days and 4 days. Figure 15(a) shows the percentage of neutrophils conjugated with platelets measured as a percentage of neutrophils expressing CD62P (% gated), and Figure 15(b) shows the percentage of neutrophils conjugated with platelets identified using CD62P median fluorescence (mf).

Examples

Example 1

For accurate research and blood diagnostics (eg blood cell conjugate studies), it is often necessary to store and preserve blood samples in such a way that best represents the *in vivo* state of the blood sample, and not the changes caused by the removal of the sample from the body's environment. This is considered to be particularly important for platelet aggregation studies. Therefore, the inventors have devised a new method according to the invention for fixing and stabilising blood cell conjugates, such as platelet aggregates, in a blood sample. The method involves mixing the blood sample with (i) a blood cell fix solution, followed by (ii) a blood cell stabiliser solution, following which the blood sample may be stored for at least 6 days. In embodiments of the invention where the method is used to stabilise platelet aggregates, following storage, the number of single platelets in the blood sample may be subsequently determined using flow cytometry, and this value may be used to determine the percentage of platelet aggregation, as described in Example 3.

Embodiments of the two solutions used in the method of the invention will now be described.

(i) Blood Cell Fix solution

The fix solution fixes single blood cells and blood cell conjugates (eg platelets and platelet aggregates) in the blood sample, and consists of 4% formalin in phosphate buffered saline (PBS), the components of which are listed in Table 1.

Table 1 - Components of the Fix solution

| Component | Concentration % (w/v) |
|---|---|
|  |  |
| (A) Buffer |  |
| Sodium chloride | 0.8% |
| Potassium chloride | 0.02 % |
| Potassium dihydrogen orthophosphate | 0.02 % |
| Disodium hydrogen orthophosphate | 0.375 % |
| Sodium dihydrogen orthophosphate monohydrate | 0.16 % |
|  |  |
| (B) Formaldehyde, paraformaldehyde or glutaraldehyde | 1.48 % |

For 100 ml, the fix solution consists of an isotonic buffered component containing the salts shown in Table 1. Once the salts have been dissolved in water, 40ml of a 10% formalin solution is added to give a final concentration of 4% of formalin. Formalin consists of about 37% formaldehyde. The solution is made up to 100ml with sterile water. The fix solution is added to blood cells at a ratio of 1 part fix solution to 3 parts blood. Hence, the concentration of the ingredients in the mixture of final blood cells/fix solution is:-

| | |
|---|---|
| Sodium chloride | 0.2 % |
| Potassium chloride | 0.005 % |
| Potassium dihydrogen orthophosphate | 0.005 % |
| Disodium hydrogen orthophosphate | 0.094 % |
| Sodium dihydrogen orthophosphate monohydrate | 0.04 % |
| Formaldehyde | 0.37% |

The fixed blood sample is stored at room temperature for 20-30 minutes to allow the fixation process to occur, ie for the blood cell conjugates (eg platelet aggregates) to be fixed. After 20-30 minutes, an aliquot of this fixed blood is then added to a tube containing the blood cell stabiliser solution.

(ii) Blood Cell Stabiliser solution

The stabiliser composition includes: (A) a buffer solution, (B) formaldehyde, and (C) a chelating agent. The components of the stabiliser composition are listed in Table 2.

Table 2 - Components of the stabiliser composition

| Component | Concentration % (w/v) |
|---|---|
| | |
| (A) Buffer solution | |
| Sodium chloride | 0.8 % |
| Potassium chloride | 0.02 % |
| Potassium dihydrogen orthophosphate | 0.02 % |
| Disodium hydrogen orthophosphate | 0.141 % |
| Sodium dihydrogen orthophosphate monohydrate | 0.016 % |
| | |
| (B) Formaldehyde, paraformaldehyde or glutaraldehyde | 0.148 % |
| | |
| (C) Chelating agent | |
| EDTA | 0.172 % |

For 100 ml, the stabiliser solution consists of an isotonic buffered component containing the five salts. Once the salts have been dissolved in water, the EDTA is then added. Formalin (37% formaldehyde) is added as a 10% solution to give a final concentration of 0.4% of formalin. The quantity of 10% formalin solution is therefore 4 ml. The solution is then made up to 100ml with sterile water. This stabiliser solution is added to blood cells that have already been fixed with the blood cell fix solution described above at a ratio of 1 part of fixed blood cell sample to 9 parts of stabiliser solution. Hence, the concentration of the ingredients in the final cells/stabiliser mixture is:

| | |
|---|---|
| Sodium chloride | 0.74% |
| Potassium chloride | 0.019% |
| Potassium dihydrogen orthophosphate | 0.019% |
| Disodium hydrogen orthophosphate | 0.136% |
| Sodium dihydrogen orthophosphate monohydrate | 0.018% |
| Ethylenediamine tetraacetic acid disodium salt dihydrate | 0.155% |

(continued)

| | |
|---|---|
| Formaldehyde | 0.170% |

Example 2

Flow cytometry

The fix and stabiliser solutions described in Example 1 may be used to stabilise any blood cell conjugate. Examples 3 to 10 describe the stabilisation of platelet aggregates, and Examples 11 to 17 describe the stabilisation of platelet-leukocyte, platelet-monocyte and platelet-neutrophil conjugates. Once stabilised, the blood sample containing the fixed conjugates may be stored for a significant period of time before being examined using flow cytometry.

Flow cytometry is a technique used for counting, examining and sorting microscopic particles suspended in a fluid stream. A beam of light is directed from a laser into a fluid stream containing the particles, and a number of detectors are aimed at the point where the stream passes through the light beam, one in line with the beam (denoted Forward Scatter or FSC) and several perpendicular to the beam (denoted Side Scatter or SSC), and one or more fluorescent detectors. Each cell that passes the laser is recorded as an "event". Each suspended particle passing through the light beam scatters the light in a certain way, and fluorescent chemicals contained in, or attached to, the particle may be excited into emitting light at a lower frequency than the light source. This combination of scattered and fluorescent light is picked up by the detectors and, by analysing fluctuations in brightness at each detector, it is possible to determine various types of information about the physical and chemical structure of each individual particle in the fluid stream.

The data generated by flow-cytometers can be plotted in a single dimension, to produce a histogram, or in two or three dimensional dot plots. The regions on these plots can be sequentially separated, based on fluorescence intensity, by creating a series of subset extractions, termed "gates". Specific gating protocols exist for diagnostic and clinical purposes, especially in relation to haematology.

Example 3

Determination of single platelet count by flow cytometric analysis

Referring to Figure 1, there are illustrated dot plots by which the number of single platelets may be quantified. Once the number of single platelets has been determined, it is then possible to calculate the percentage degree of platelet aggregation. Quantification of single platelets in whole blood by flow cytometry required 50,000 red cell events which were collected in region P1 on a forward scatter/side scatter plot, as shown in Figure 1 a. CD42a fluorescence was plotted against forward scatter, and region P2 was selected by drawing a curved line to include all platelet-related events and excluding erythrocyte/platelet co-incidences marked "e/p", as shown in Figure 1 b. CD42a-positive platelet events in P2 were then redrawn, as shown in Figure 1 c. EDTA-anti-coagulated blood, or citrate anti-coagulated blood containing EDTA thus containing unstimulated platelets, was used to set three rectangular gates to quantitate microparticles (P3), single platelets (P4), and microaggregates (P5). These gates were then applied to the dot plots obtained for stimulated samples of blood, recording single platelets in P4. An example of results from an ADP-stimulated, fixed sample in which the single platelet events (P4) reduced from 1716 to 182 (indicating the formation of platelet aggregates) is shown in Figures 1d, 1e and 1f.

Fixed and stabilised blood samples were mixed gently by repeated inversion of the sample tubes. 100$\mu$l of the fixed blood was placed in a flow cytometry tube and centrifuged at 380g for 5 minutes after which 90$\mu$l of the supernatant is removed. The remaining 10$\mu$l of fixed blood was then incubated with 10 $\mu$l of a 1 in 10 dilution of CD42a-FITC-labelled antibody for 20 min at 4°C in the FACS tubes. 1 ml of FACSflow (or any other flow cytometry diluent) was added to the tubes immediately prior to reading on any flow cytometer equipped with a laser operating at an excitation wavelength of 488nm and with detectors for measurement at suitable wavelengths for the selected fluorochromes (ie FITC). Appropriate software was used to acquire and analyse data on dot plots of forward scatter versus side scatter and forward scatter versus FITC fluorescence acquired with logarithmic amplification. Acquisition was stopped after recording 50,000 erythrocyte events. A sample of fixed unstimulated EDTA-anti-coagulated blood was used to set the vertical lines of three square gates P3, P4 and P5 on a forward scatter versus FITC fluorescence plot. These gates were adjusted to record microparticles in P3, single platelets in P4 and any microaggregates in P5 (see Example 2). The test samples are then analysed using the same gates. Larger aggregates would not be detected by the flow cytometer since they would be too large and cannot pass through the sample tube of the machine.

Antibody to CD42a conjugated to FITC was used in the experiments. However, platelet-specific antibodies other than CD42a (eg CD61) may also be used, and fluorochromes other than FITC may be substituted provided that they are found to be suitable for the analysis of fixed samples. Appropriate assessment by an experienced flow cytometry expert of the suitability of any other platelet-specific antibody or any other fluorescent probe would be necessary before their use in the analysis of these fixed samples.

Calculation of % aggregation from the single platelet count

The single platelet count described above is a record of the number of single platelets present in each blood sample (recorded as single platelet events, ie the number of single platelets passing the laser) in relation to 50,000 erythrocyte events recorded, ie erythrocytes passing the laser. It is possible to calculate a true platelet count by using standard flow cytometry counting beads, such as those sold under the trade name Trucount. Aggregation is usually determined as a percentage, and in whole blood this may be recorded as a percentage fall in single platelets. Hence, as the number of single platelets decreases, the number of platelet aggregates must be increasing. It is therefore possible to calculate percentage aggregation directly from the recordings of single platelet events using the following formula:-

$$\% \text{ aggregation} = \frac{100 \times [\text{EDTA count-Sample count}]}{[\text{EDTA count}]}$$

Where "EDTA count" is the number of platelet events (ie single platelets passing the laser) for a sample containing EDTA which has been fixed in the same way as the test samples, and where "Sample count" is the number of platelet events for the fixed test sample.

Example 4

Figure 2 shows various samples of dot plots used to determine the number of platelet events in a fixed blood sample using the quantification method described above. A sample of fixed and stabilised citrate anti-coagulated blood containing EDTA (which prevents aggregation, ie 0% aggregation) and thus unstimulated, non-aggregated platelets was used to set three rectangular gates P3, P4 and P5 as previously described, to determine the number of platelet events in P4, as shown in Figure 2a. A sample of blood that had been stirred for 4 minutes with the platelet stimulant collagen (1μg/ml) was fixed and stabilised, and the number of single platelet events in P4 recorded, as shown in Figure 2b. Aggregation in the presence of collagen was calculated as a percentage fall in single platelets compared with the blood sample containing EDTA (0% aggregation). The "EDTA count" was 1118, and the "sample count" was 693. Hence, using the formula in Example 3, the degree of aggregation is calculated as about 38%.
Further examples of dot plots were then used to determine the number of platelet events in fixed and stabilised blood samples from the same donor, but stimulated witch either 3μM Platelet Activating Factor (PAF) (as shown in Figure 2c) or 10μM Thrombin Receptor Activating Peptide (TRAP) (as shown in Figure 2d). Aggregation was calculated from the same EDTA result (obtained in Figure 2a) as a percentage fall in single platelets. The degree of aggregation caused by the stimulant PAF was 70%, whereas the stimulant TRAP caused 89% aggregation.
Further examples of dot plots were used to determine the number of platelet events in fixed and stabilised blood samples from the same donor but stimulated with either 200nM arachidonic acid (AA) (as shown in Figure 2e) or 1μM U46619 (as shown in Figure 2f). Aggregation was calculated from the same EDTA result (obtained in Figure 2a) as a percentage fall in single platelets. The degree of aggregation caused by the stimulant AA was 54%, whereas the stimulant U46619 caused 96% aggregation.
From these data, it is clear that the amount of platelet aggregation may be easily determined using the fix and stabiliser solutions in the method of the invention, followed by flow cytometry.

Example 5

Figure 3 shows an example of an aggregation curve measured in fixed and stabilised citrate-anti-coagulated blood samples previously stirred with a range of concentrations of the platelet stimulant, ADP. The number of single platelet events was determined, as previously, using the quantification procedure described in Example 1. A sample of fixed and stabilised citrate anti-coagulated blood containing EDTA and thus unstimulated, non-aggregated platelets, was used to set three rectangular gates P3, P4 and P5 (not shown) and to determine the number of single platelet events in P4 as a starting platelet count. Samples of citrate anti-coagulated blood were also stirred for 4 minutes with saline (0) and 1 μM, 3 μM and 10 μM of the platelet activator ADP, and then fixed and stabilised. Aggregation was calculated as a percentage fall in single platelets. From Figure 3, it can be seen that the EDTA-containing control sample contained 0% platelet aggregation as expected, and that 1 μM ADP-containing samples exhibited 27% aggregation, 3μM ADP-containing samples exhibited 62% aggregation, and that 10μM ADP-containing samples exhibited 89% aggregation.

Hence, the inventors have demonstrated that the method of the invention can be used to reliably stabilise the platelet aggregation induced by varying concentrations of the activator ADP.

Example 6

Referring to Figure 4, there is shown the results of platelet aggregation measurements over 9 days in citrate-anti-coagulated blood following stimulation with a range of collagen (a platelet activator) concentrations (1, 2, 3, and 4 $\mu$g/ml) for 10 minutes in the absence (left) or presence (right) of the anti-thrombotic agent, aspirin. Results were obtained from three different donors, Donor 1 to Donor 3. As can be seen, aspirin, which is a known anti-thrombotic agent, and which prevents platelet activation, had the effect of significantly reducing the amount of aggregation occurring in the sample, even in the presence of the platelet stimulant collagen. Furthermore, the inventors were surprised to observe that, for each concentration of stimulant, there was a negligible decrease in the amount of aggregation occurring over the 9-day test period. This demonstrates the efficacy of the fix and stabiliser solutions and the method of the invention.

Example 7

Referring to Figure 5, there is shown the results of platelet aggregation measurements over 9 days in citrate-anti-coagulated blood following stimulation with a range of concentrations of the platelet stimulant ADP (0.3-10$\mu$M), following stirring for 1 minute (left) and 4 minutes (right) in order to obtain a wide range of aggregation values. Results were obtained from three different donors. The inventors were surprised to observe that, for each concentration of ADP stirred for each time point, there was a negligible decrease in the amount of aggregation recorded over the 9-day test period.

Example 8

Referring to Figure 6, there is shown the results of platelet aggregation measurements over 9 days in citrate-anti-coagulated blood following stimulation with saline (control), Platelet Activating Factor (PAF), Sulprostone (Sulp), or a combination of both stimulants (P&S), for 10 minutes. Results were obtained from three different donors. The inventors were surprised to observe that, for each stimulant or combination of stimulants, there was a negligible decrease in the amount of aggregation occurring over the 9-day test period. This demonstrates the efficacy of the fix and stabiliser solutions and the method of the invention.

Example 9

Referring to Figure 7, there is shown the results of aggregation measurements over 9 days in citrate-anti-coagulated blood following stimulation with ADP, a combination of ADP and Sulprostone (ADP S), U46619 (U4) and prostaglandin E2 (U4 E2) or U46619 and Sulprostone (U4 S), for 4 minutes. Results were obtained from six different donors. Clearly, there is only a negligible decrease in the degree of platelet aggregation over the 9-day test period for any of the stimulants tested. Accordingly, the inventors have demonstrated that the method of the invention may be efficiently used to reliably stabilise platelet aggregates in blood samples for at least 9 days. Once fixed and stabilised by the method of the invention, samples may then be transported long distances, if necessary, to remote blood diagnostic facilities where they may be tested by flow cytometry. With this in mind, the inventors developed a protocol for a platelet aggregation kit as described in Example 10.

Example 10

Figure 8 shows an example of a protocol for measuring whole blood aggregation using the method and kits of the invention. Referring to Figure 9, there is shown a multi-sample agitator (MSA), which is designed for blood cell conjugate studies, such as platelet aggregation, and is manufactured by the University of Nottingham. The MSA is a portable, compact piece of equipment that is designed to agitate blood samples in a reproducible and consistent way. It consists of a temperature-controlled aluminium block with a magnetic stirring device, which provides reliable accurate temperature control and stirring with no potential for water leaks or risk of bacterial contamination. Twelve evenly spaced wells, located in the central portion of the block in a circular arrangement, are designed to accommodate standard 64 x 11 mm polystyrene tubes in which 500 $\mu$l aliquots of blood are placed. A small PTFE-coated magnetic flea is placed in the blood and agitated via a magnetic stirring device located beneath the aluminium block. For platelet studies, the temperature of the aluminium block is set at 37°C and the degree of agitation is set at 1000 rpm. The block also has 17 additional wells arranged in an outer circle for pre-incubation of blood and storage. These consist of a further 12 wells of the same size as the central wells together with a further 5 wells which can accommodate larger 95 x 16.8mm capped polystyrene tubes containing up to 10ml of anti-coagulated blood.

With reference to Figure 8, polystyrene aggregation tubes are pre-filled with a small volume (eg 20µl) of a platelet stimulant (eg ADP, TRAP, collagen, epinephrine, the thromboxane mimetic U46619, or arachidonic acid etc), as required before the kit is used. These stimulants may be varied according to the requirements of the test but should be able to activate the platelets, and therefore promote platelet aggregation. The concentration of each stimulant is pre-determined, and designed to give optimal aggregation of the blood. A small magnetic stirrer bar may also be added to each tube. Additionally, other substances such as known platelet inhibitors or enhancers can also be added to the stimulant tubes. Furthermore, in some embodiments of the kit, it may be desirable to add an anti-coagulant (eg sodium citrate or hirudin) to the blood sample. Therefore, the kit includes a tube containing an anti-coagulant, such as citrate or hirudin.

After a 30 minute incubation of the blood sample in the anti-coagulation agent, a 480µl aliquot of blood is then dispensed into the tube containing the platelet stimulant, shown as step A in Figure 8. The stimulant tubes are agitated by shaking or stirring at 1000 rpm in the multi-sample agitator shown in Figure 9 for a pre-determined time (eg about 4 minutes at 37°C) in order for full stimulation of the platelets to take place. Enough agitation is required to allow platelet-platelet contact to occur and platelet aggregates to form. However, agitation should not be so aggressive that either cell aggregates cannot form, or so that they are broken up once formed. Following platelet stimulation in step A, 167µl of the fix solution is then added to each stimulant tube at pre-determined time intervals, shown as step B in Figure 8. As a control, if desired, the fix solution can be added to a separate blood sample immediately after venepuncture to stabilise the blood and prevent any aggregate formation in the absence of any anti-coagulant or stimulant. The control tube is used to test the blood sample for any basal levels of platelet aggregation. In addition, a control tube containing EDTA instead of platelet stimulant may be fixed and stabilised in the same way to provide a count of non-aggregated, single platelets.

After 30 minutes of exposure to the fix solution at room temperature, 150µl of fixed blood sample from each aggregation tube is added to a corresponding microtube containing 1350µl of the stabiliser solution, and then capped and thoroughly mixed, shown as step C in Figure 8. The blood sample is thus fixed (by the fix solution which contains a high concentration of formaldehyde in buffer) and stabilised (by the stabiliser solution which contains a lower concentration of formaldehyde with EDTA in buffer), and may be stored for many days prior to subsequent analysis of the degree of platelet aggregation. In some embodiments of the kit and protocol, the volumes of the aliquots may be designed so that the stabiliser solution may be added directly to the fixed blood rather than adding an aliquot of the fixed blood to the stabiliser solution.

It should be appreciated that Steps A-C can be performed at room temperature, at 37°C, or at other temperatures as required for a fixed time period (eg 4 minutes) or at pre-determined time intervals (eg 30s, or 1, 2, 4, 8, 10 minutes) in order to obtain an aggregation-time curve. The fixed and stabilised samples may then be stored at either room temperature or at lower, chilled temperatures (eg 4°C) to await flow cytometric analysis, as described in Examples 2 and 3.

Example 11

In addition to platelet aggregates, platelets may also conjugate with leukocytes. The formation of platelet-leukocyte conjugates may occur in the blood *in situ* following platelet activation and measurement of basal levels of circulating conjugates may provide an indication of this. Blood may be fixed and stabilised immediately after venepuncture to assess basal levels of platelet-leukocyte conjugate formation. In addition, *in vitro* addition of stimulants to anticoagulated, stirred blood will induce platelet-leukocyte conjugate formation and this may also be determined in blood fixed and stabilised using the invention.

Platelet- leukocyte conjugates are measured using different flow cytometric procedures and templates to those employed to measure platelet aggregation. These procedures involve the use of leukocyte- identifying markers, such as CD14, which is specifically expressed on monocytes, or other markers, such as the leukocyte marker CD45, may be used to identify the different types of leukocyte present in the blood samples. In addition, specific platelet markers such as CD42a, CD61 or CD41 may be used to identify the presence of platelets. When both types of markers are co-expressed on one event, then this would indicate that a conjugate of both platelets and leukocytes is present. Fixed and stabilised blood is incubated with appropriate antibodies and then analysed using an appropriate flow cytometer.

For example, fixed and stabilised samples are removed from the fridge and mixed well until the sedimented cells are fully re-suspended. 500µl of each sample is then dispensed into appropriately labelled flow cytometry tubes and centrifuged for 10min at 380g. 450µl of the supernatant is then removed, taking care not to disturb the red blood cell pellet. 10µl of saturating concentrations of CD14-APCCy7 (used to distinguish monocytes and neutrophils) and CD42a-FITC or CD62P-PE (used to identify platelets bound to either monocytes or neutrophils) is then added to the tubes and incubated with the cells in the dark. After 25 minutes, 1 ml of Erythrolyse solution (AbD Serotec, Oxford, UK) is added to the cells and left to stand for 10 minutes to lyse any red blood cells. 1ml of FACSflow is added and the cells centrifuged at 380g. Supernatants are decanted and cells re-suspended in 2ml of FACSflow to wash the cells. The cells are then centrifuged at 380g again, the supernatant removed and the cells finally re-suspended in $300\mu l$ of FACSflow to await reading on the flow cytometer.

Leukocytes are initially identified by logical gating from dot plots of forward scatter (cell size) and side scatter (cell granularity) profiles acquired with linear amplification. Fluorescence parameters are acquired with logarithmic amplification and 30,000 leukocyte events are collected for each blood sample. The monocytes are then identified by their forward scatter/side scatter profile and CD14 positivity, while neutrophils are identified in the same way but are negative for CD14 expression with high forward scatter/side scatter profiles. Lymphocytes may also be identified by CD14 negativity and a low forward scatter/side scatter profile, but platelet-lymphocyte conjugates are formed to a lesser extent and are not routinely quantitated. Platelet-monocyte and platelet-neutrophil conjugates are quantified as the percentage of monocytes or neutrophils expressing platelet CD42a or CD62P fluorescence in an interval gate which may be adjusted using an appropriate IgG control in each case. Median fluorescence values for CD42a or CD62P are also measured for each population of leukocytes tested. Figures 10, 11, and 12 show flow cytometry data for platelet-monocyte and platelet-neutrophil conjugates.

Example 12

Referring to Figure 10, there are shown dot plots used for the identification of platelet-leukocyte conjugates in fixed and stabilised blood samples following incubation with (a) saline, or (b) collagen. Dot plots of (i) forward-scatter versus side scatter, (ii) CD14-APCCy7 versus side scatter, and (iii) CD42a-FITC versus CD14-APCCy7 were prepared. Using plot (ii), gates were drawn around the CD14-positive monocytes (P2) and CD14-negative neutrophils (P1). Using plot (iii), rectangle gates were drawn around the CD14-positive monocytes (P3) and CD14-negative neutrophils (P4). Monocytes were therefore identified by their co-appearance in P2 and P3, while neutrophils were identified by co-appearance in P1 and P4. Platelet-monocyte and platelet-neutrophil conjugate formation following stimulation with collagen is seen as a shift to the right in plot (iii) as the degree of platelet-specific CD42a fluorescence (ie marker expression) increases.

Example 13

Referring to Figure 11, there is shown the quantification of platelet-monocyte conjugates (identified from gates P2 and P3 as shown in Figure 10) which are detected with the platelet-specific markers CD42a or CD62P. Quantification is detected as a percentage shift of the parent monocyte population from the bottom right to top right quadrants (Q2 and Q2-1) as more platelets are detected on the monocytes via their expression of CD42a or CD62P (% gated), and as the median fluorescence values (mf) of the monocyte population (P2 and P3) for CD42a or CD62P.

Figure 11(i) shows CD42a quantification of platelet-monocyte conjugates after incubation with (a) saline or (b) collagen. Following collagen stimulation, the proportion of conjugates increases from 8.2 to 64.1 % of the parent population, and CD42a expression, as evidenced by median fluorescence (arbitrary units), increases from 259 to 832. Figure 11(ii) shows CD62P quantification of platelet-monocyte conjugates after incubation with (a) saline or (b) collagen. Following collagen stimulation, the proportion of conjugates increases from 9.3 to 98.7% of the parent population, and CD62P expression, as evidenced by median fluorescence, increases from 375 to 2241.

Example 14

Referring to Figure 12, there is shown the quantification of platelet-neutrophil conjugates (identified from gates P1 and P4 as shown in Figure 10) which are detected with the platelet-specific markers CD42a or CD62P. Quantification is detected as a percentage shift of the parent neutrophil population (P1+P4) from the bottom right to top right quadrants (Q2-3 and Q2-4) as either more platelet CD42a or CD62P is detected on the neutrophils (% gated), and as the median fluorescence values (mf) of the neutrophil population (P1 and P4) for CD42a or CD62P.

Figure 12(i) shows CD42a quantification of platelet-neutrophil conjugates after incubation with (a) saline or (b) collagen. Following collagen stimulation, the proportion of conjugates increases from 7.9 to 21.9% of the parent population, and CD42a expression, as evidenced by median fluorescence increases from 142 to 201. Figure 12(ii) shows CD62P quantification of platelet-neutrophil conjugates after incubation with (a) saline or (b) collagen. Following collagen stimulation, the proportion of conjugates increases from 7.3 to 72.1% of the parent population, and CD62P expression, as evidenced by median fluorescence increases from 141 to 688.

Example 15

Aliquots of the same fixed and stabilised blood samples as those prepared for measuring platelet aggregation were used to measure platelet-leukocyte conjugate formation. Using these fixed and stabilised samples prepared with the method according to the invention, levels of platelet-monocyte and platelet-neutrophil conjugates were measured in fixed and stabilised samples that have been stored at 4°C for up to 4 days. Examples are shown of platelet-monocyte conjugates that have been detected using a combination of CD14 and CD42a, or a combination of CD14 and CD62P. An example of platelet-neutrophil conjugates that were detected with CD14 and CD62P are also described below.

The inventors wished to investigate the efficacy of the method of the invention for fixing and stabilising blood samples for measuring platelet-monocyte conjugates using CD14 and CD42a. Therefore, the stability of fixed and stabilised blood samples obtained from three separate donors that had been stimulated at 37 °C with a range of concentrations of the platelet stimulant collagen or with saline alone, in the presence or absence of aspirin (ASP), was then assessed. Platelet-monocyte conjugate formation was measured within 5 minutes of fixing and stabilising the stirred blood with the compositions of the invention, and then again after storage at 4°C for 2 days and 4 days using the same procedures described in Example 13 and Figure 11(i). The results are shown in Figure 13 as (a) the percentage of monocytes expressing CD42a (% gated), or (b) using CD42a median fluorescence (mf). The inventors were pleased to observe that the expression of CD42a remained constant over the whole 4 days of the experiment. This is considered to be a significant advantage when using the methods of the invention for stabilising platelet-monocyte conjugates. This is because someone wishing to analyse the level of platelet-monocyte conjugates in a blood sample can be confident that testing for CD42a expression would give a true value if tested as long as 4 days after taking the sample.

Example 16

The inventors wished to investigate the efficacy of the fixed and stabilised blood samples for measuring platelet-monocyte conjugates using CD14 and CD62P. Therefore, the stability of fixed and stabilised blood samples obtained from three separate donors that had been stimulated at 37°C with a range of concentrations of the platelet stimulant collagen or with saline alone, in the presence or absence of aspirin (ASP), was then assessed. Platelet-monocyte conjugate formation was measured within 5 minutes of fixing and stabilising the stirred blood with the compositions of the invention and then again after storage at 4°C for 2 days and 4 days using the same procedures described in Example 13 and Figure 11(ii). The results are shown in Figure 14 as a percentage of monocytes conjugated with platelets measured as (a) the percentage of monocytes expressing CD42a (% gated), or (b) using CD62P median fluorescence (mf). The inventors were pleased to observe that the expression of CD62P remained constant over the whole 4 days of the experiment. This is considered to be a significant advantage when using the methods of the invention for stabilising platelet-monocyte conjugates.

Example 17

The inventors wished to investigate the efficacy of the method of the invention for fixing and stabilising blood samples for measuring platelet-neutrophil conjugates using CD14 and CD62P. Therefore, the stability of fixed and stabilised blood samples obtained from three separate donors that had been stimulated at 37°C with a range of concentrations of the platelet stimulant collagen or with saline alone, in the presence or absence of aspirin (ASP), was then assessed. Platelet-neutrophil conjugate formation was measured within 5 minutes of fixing and stabilising the stirred blood with the compositions of the invention and then again after storage at 4°C for 2 days and 4 days using the same procedures described in Example 14 and Figure 12(ii). The results are shown in Figure 15 as a percentage of neutrophils conjugated with platelets measured as (a) the percentage of neutrophils expressing CD62P (% gated), or (b) using CD62P median fluorescence (mf). The inventors were pleased to observe that the expression of CD62P remained constant over the whole 4 days of the experiment. This is considered to be a significant advantage when using the methods of the invention for stabilising platelet-neutrophil conjugates.

Example 18

The inventors have developed a kit which may be used to assess the antithrombotic efficacy of a $P2Y_{12}$ antagonist such as clopidogrel which reduces platelet activation. This test may be used to check the effectiveness of a $P2Y_{12}$ antagonist even during co-administration of aspirin which inhibits platelet function via a different mechanism (inhibition of COX-1). Blood samples were obtained from a group of healthy volunteers (a) before and (b) after treatment with clopidogrel. Blood samples were also obtained (c) before and (d) after treatment with a combination of both clopidogrel and aspirin. The blood samples were anticoagulated with sodium citrate and small aliquots added to stimulant tubes containing either EDTA or ADP 10$\mu$M (as described in Example 10 on page 45) and fixed and stabilised (as described in Example 10 on page 46) and analysed by flow cytometry (as described in Example 3 on pages 38-40). Numbers of platelet events were determined using antibody to CD42a and platelet aggregation was calculated as a percentage of the EDTA sample (see Figures 2a-f).

When platelet aggregation was measured in the group of volunteers prior to treatment with clopidogrel a high amount of aggregation was obtained after addition of blood to the stimulant tube containing ADP. When this test was repeated following clopidogrel administration less aggregation was seen demonstrating the inhibitory effect of the clopidogrel. When platelet aggregation was measured in the group of volunteers prior to treatment with the combination of clopidogrel and aspirin a high aggregation was again obtained after addition of blood to the ADP stimulant tube. When the test was repeated on a blood sample obtained following administration of clopidogrel and aspirin much less aggregation was recorded though the clopidogrel test was not affected by co-administration of aspirin.

Hence, it can be seen from Figure 16 that platelet aggregation was inhibited in both groups following administration of clopidogrel even when aspirin was present.

This data demonstrates that the composition of the invention may be used to stabilise activated blood samples for subsequent measurement of platelet aggregation in a kit designed to assess the anti-thrombotic efficacy of $P2Y_{12}$ antagonists such as clopidogrel.

**Claims**

1.    A method of stabilising blood cell conjugates in a sample, wherein blood cell conjugates comprise two or more blood cells joined together wherein the blood cells are one or more of platelets, erythrocytes and leukocytes, the method comprising the steps of:

    (i) contacting a sample containing blood cells with a first composition comprising an aliphatic aldehyde and a buffer, to thereby fix blood cell conjugates present in the sample such that any blood cell conjugates present in the sample remain together and cannot fall apart, and any single cells remain unconjugated, either before or during subsequent analysis; and

    (ii) contacting the sample fixed in step (i) with a second composition comprising an aliphatic aldehyde, a chelating agent, and a buffer to thereby stabilise the blood cell conjugates, wherein the concentration of aliphatic aldehyde in contact with the blood cells after contact with the second composition is less than after step (i).

2.    A method as claimed in Claim 1, wherein the method comprises the step of contacting the sample with an anticoagulant prior to contacting with the first composition in step (i).

3.    A method as claimed in any preceding claim, wherein step (i) is conducted in the absence of ethylenediaminetetraacetic acid (EDTA).

4.    A method as claimed in any preceding claim, wherein the aliphatic aldehyde in the first composition and/or in the second composition is glutaraldehyde or formaldehyde.

5.    A method as claimed in any preceding claim, wherein the concentration of the aliphatic aldehyde in the first composition is at least 0.1% (v/v), preferably at least 0.5% (v/v), more preferably at least 1%(v/v).

6.    A method as claimed in any preceding claim, wherein the concentration of the aliphatic aldehyde in the first composition is between about 0.1 and 15% (v/v), preferably between about 0.5 and 10% (v/v), and most preferably between about 1 and 5% (v/v).

7.    A method as claimed in any preceding claim, wherein the buffer in the first composition is selected from the group consisting of phosphate buffered saline (PBS), isotonic saline, Tris, N-(2-acetamido)-2-iminodiaacetic acid, and buffers prepared from pyrophosphate, 4-(2-hydroxyethyl)-1-piperazineethanesulfonic acid (HEPES), acetate, imidazole, succinate, maleate, citrate, carbonate, methylethyl sulfide (MES), 3-(N-morpholino) propanesulfonic acid (MOPS), and combinations thereof.

8.    A method as claimed in any preceding claim, wherein the sample containing blood cells remains in contact with the first composition for between about 10 min and 40 minutes before addition of the second composition in step (ii).

9.    A method as claimed in any preceding claim, wherein the concentration of aliphatic aldehyde in the second composition is between 0.01 and 5% (v/v), preferably between 0.03 and 1% (v/v), more preferably between 0.05 and 0.5% (v/v), and most preferably between 0.1 and 0.2% (v/v).

10.    A method as claimed in any preceding claim, wherein the total amount of aliphatic aldehyde in the combined composition in step (ii) is between 0.05 and 0.5% (v/v), preferably between 0.1 and 0.2% (v/v).

11. A method as claimed in any preceding claim, wherein the concentration of aliphatic aldehyde in the first composition is higher than the concentration of aliphatic aldehyde in the second composition.

12. A method as claimed in Claim 11, wherein the concentration of aliphatic aldehyde in the first composition is at least double the concentration of aliphatic aldehyde in the second composition, more preferably at least 5 times greater, more preferably at least 8 times greater, and most preferably the concentration of aliphatic aldehyde in the first composition is about 10 times greater than the concentration of aliphatic aldehyde in the second composition.

13. A method as claimed in any preceding claim, wherein the chelating agent in the second composition is selected from the group consisting of ethylenediaminetetraacetic acid (EDTA), ethyleneglycol-bis($\beta$-aminoethylether)-*N,N, N',N'*-tetraacetic acid (EGTA), trans-1,2-diamino-cyclohexane-*N,N,N',N'*-tetraacetic acid (CDTA), nitriloacetic acid (NTA), Porphine, Heme, 1,2-bis(o-aminophenoxy)ethane-*N,N,N',N'*-tetraacetic acid (BAPTA), Dimercaprol, and combinations thereof.

14. A method as claimed in any preceding claim, wherein the blood cell conjugate is a platelet aggregate.

15. A blood cell conjugate stabilisation kit, the kit comprising a first container in which a first composition comprising an aliphatic aldehyde and a buffer is contained, and a second container in which a second composition comprising an aliphatic aldehyde, a chelating agent and a buffer is contained, and optionally instructions for use; wherein the concentration of aliphatic aldehyde in the first composition is higher than the concentration of aliphatic aldehyde in the second composition.

**Patentansprüche**

1. Ein Verfahren zur Stabilisierung von Blutzellkonjugaten in einer Probe, worin die Blutzellkonjugate zwei oder mehr Blutzellen enthalten, die miteinander verbunden sind, wobei die Blutzellen eines oder mehrere von Thrombozyten, Erythrozyten und Leukozyten sind, und wobei das Verfahren die folgenden Schritte umfasst:

(i) in Kontakt Bringen einer Probe, die Blutzellen enthält, mit einer ersten Zusammensetzung, die aus einem aliphatischen Aldehyd und einem Puffer besteht, um auf diese Weise die in der Probe vorhandenen Blutzellkonjugate zu fixieren, sodass entweder vor oder nach der darauffolgenden Analyse alle in der Probe vorhandenen Blutzellkonjugate zusammen bleiben und nicht auseinanderfallen können, und all Einzelzellen unkonjugiert bleiben; und

(ii) in Kontakt bringen der in Schritt (i) fixierten Probe mit einer zweiten Zusammensetzung, die ein aliphatisches Aldehyd, einen Chelatbildner und einen Puffer enthält, um auf diese Weise die Blutzellkonjugate zu stabilisieren, wobei die aliphatische Aldehydkonzentration, die nach Kontakt mit der zweiten Zusammensetzung in Kontakt mit den Blutzellen kommt, niedriger ist als nach Schritt (i).

2. Ein Verfahren, wie in Anspruch 1 beansprucht, worin das Verfahren den Schritt des Inkontaktbringens der Probe mit einem Antikoagulans vor dem Inkontaktbringen mit der ersten Zusammensetzung in Schritt (i) beinhaltet.

3. Ein Verfahren, wie in einem vorhergehenden Anspruch beansprucht, worin Schritt (i) in Abwesenheit von Ethylendiamintetraessigsäure (EDTA) durchgeführt wird.

4. Ein Verfahren, wie in einem vorhergehenden Anspruch beansprucht, worin das aliphatische Aldehyd in der ersten Zusammensetzung und/oder in der zweiten Zusammensetzung Glutaraldehyd oder Formaldehyd ist.

5. Ein Verfahren, wie in einem vorhergehenden Anspruch beansprucht, worin die aliphatische Aldehydkonzentration in der ersten Zusammensetzung mindestens 0,1% (v/v), vorzugsweise mindestens 0,5% (v/v) und am besten mindestens 1 % (v/v) beträgt.

6. Ein Verfahren, wie in einem vorhergehenden Anspruch beansprucht, worin die aliphatische Aldehydkonzentration in der ersten Zusammensetzung zwischen ca. 0,1 und 15% (v/v), vorzugsweise zwischen 0,5 und 10% (v/v) und am besten zwischen ca. 1 und 5% (v/v) beträgt.

7. Ein Verfahren, wie in einem vorhergehenden Anspruch beansprucht, worin der Puffer in der ersten Zusammensetzung ausgewählt wird aus der Gruppe, bestehend aus Phosphat-gepufferter Kochsalzlösung (PBS), Isotonischer Kochsalzlösung, Tris, N-(Acetamido)-2-Iminodiessigsäure und Puffern, die aus Pyrophosophat, 4-(2-Hydroxyethyl)-1-Piperazineethansulfonsäure (HEPES), Acetat, Imdiazol, Succinat, Maleat, Citrat, Carbonat, Methylethylsulfid (MES), 3-(N-Morpholino)-Propansulfonsäure (MOPS) und Kombinationen daraus hergestellt werden.

8. Ein Verfahren, wie in einem vorhergehenden Anspruch beansprucht, worin die Probe, welche Blutzellen enthält, zwischen ca. 10 min und 40 min vor der Zugabe der zweiten Zusammensetzung in Schritt (ii) in Kontakt mit der ersten Zusammensetzung bleibt.

9. Ein Verfahren, wie in einem vorhergehenden Anspruch beansprucht, worin die alphatische Aldehydkonzentration in der zweiten Zusammensetzung zwischen 0,01 und 5% (v/v), vorzugsweise zwischen 0,03 und 1 % (v/v), noch besser zwischen 0,05 und 0,5% (v/v) und am besten zwischen ca. 0,1 und 0,2% (v/v) beträgt.

10. Ein Verfahren, wie in einem vorhergehenden Anspruch beansprucht, worin die Gesamtmenge von aliphatischem Aldehyd in der kombinierten Zusammensetzung in Schritt (ii) zwischen 0,05 und 0,5% (v/v) und vorzugsweise zwischen 0,1 und 0,2% (v/v) beträgt.

11. Ein Verfahren, wie in einem vorhergehenden Anspruch beansprucht, worin die aliphatische Aldehydkonzentration in der ersten Zusammensetzung höher ist als die alphatische Aldehydkonzentration in der zweiten Zusammensetzung.

12. Ein Verfahren, wie in Anspruch 11 beansprucht, worin die aliphatische Aldehydkonzentration in der ersten Zusammensetzung mindestens das Doppelte der alphatischen Aldehydkonzentration in der zweiten Zusammensetzung beträgt, noch besser mindestens 5 mal so groß ist, noch besser mindestens 8 mal so groß ist und die aliphatische Aldehydkonzentration in der ersten Zusammensetzung am besten 10 mal so groß ist wie die alphatische Aldehydkonzentration in der zweiten Zusammensetzung.

13. Ein Verfahren, wie in einem vorhergehenden Anspruch beansprucht, worin der Chelatbildner in der zweiten Zusammensetzung ausgewählt wird aus der Gruppe bestehend aus Ethylendiamintetraessigsäure (EDTA), Ethylenglycolbis(β-aminoethylether)-*N,N,N',N'*-Tetraessigsäure (EGTA), Trans-1,2-diamin-cyclohexan-*N,N,N'N'*-Tetraessigsäure (CDTA), Nitriloessigsäure (NTA), Porphin, Häm, 1,2-bis(o-Aminophenoxy)ethan-*N,N,N',N'*-Tetraessigsäure (BAPTA), Dimercaprol und Kombinationen davon.

14. Ein Verfahren, wie in einem vorhergehenden Anspruch beansprucht, worin das Blutzellkonjugat ein Thrombozytenaggregat ist.

15. Ein Blutzellkonjugat-Stabilisierungskit, wobei das Kit einen ersten Behälter umfasst, der eine erste Zusammensetzung beinhaltet, welche ein aliphatisches Aldehyd und einen Puffer enthält, und einen zweiten Behälter, der eine zweite Zusammensetzung beinhaltet, welche ein aliphatisches Aldehyd, einen Chelatbildner und einen Puffer enthält, sowie wahlweise eine Gebrauchsanweisung; worin die aliphatische Aldehydkonzentration in der ersten Zusammensetzung höher ist als die aliphatische Aldehydkonzentration in der zweiten Zusammensetzung.

## Revendications

1. Un procédé de stabilisation de conjugués de globules sanguins dans un échantillon, où les conjugués de globules sanguins contiennent deux ou plus globules sanguins joints ensemble, où les globules sanguins sont un élément ou plusieurs parmi plaquettes, érythrocytes et leucocytes, le procédé comprenant les opérations suivantes :

(i) la mise en contact d'un échantillon contenant des globules sanguins avec une première composition contenant un aldéhyde aliphatique et un tampon, de façon à ainsi fixer des conjugués de globules sanguins présents dans l'échantillon de sorte que tous les conjugués de globules sanguins présents dans l'échantillon demeurent ensemble et ne puissent pas se séparer, et toutes les cellules uniques demeurent non conjuguées, soit avant ou au cours d'une analyse subséquente, et
(ii) la mise en contact de l'échantillon fixé à l'opération (i) avec une deuxième composition contenant un aldéhyde aliphatique, un agent chélateur et un tampon de façon à ainsi stabiliser les conjugués de globules sanguins, où la concentration d'aldéhyde aliphatique en contact avec les globules sanguins après contact avec la deuxième composition est plus faible que celle après l'opération (i).

**2.** Un procédé selon la Revendication 1, où le procédé comprend l'opération de mise en contact de l'échantillon avec un anticoagulant avant la mise en contact avec la première composition à l'opération (i).

**3.** Un procédé selon l'une quelconque des Revendications précédentes, où l'opération (i) est réalisée en l'absence d'acide éthylène diamine tétraacétique (EDTA).

**4.** Un procédé selon l'une quelconque des Revendications précédentes, où l'aldéhyde aliphatique dans la première composition et/ou dans la deuxième composition est glutaraldéhyde ou formaldéhyde.

**5.** Un procédé selon l'une quelconque des Revendications précédentes, où la concentration de l'aldéhyde aliphatique dans la première composition est au moins de 0,1% (v/v), de préférence au moins de 0,5% (v/v), de préférence encore au moins de 1% (v/v).

**6.** Un procédé selon l'une quelconque des Revendications précédentes, où la concentration de l'aldéhyde aliphatique dans la première composition se situe entre environ 0,1 et 15% (v/v), de préférence entre environ 0,5 et 10% (v/v), et idéalement entre environ 1 et 5% (v/v).

**7.** Un procédé selon l'une quelconque des Revendications précédentes, où le tampon dans la première composition est sélectionné dans le groupe se composant de solution saline tamponnée au phosphate (PBS), solution saline isotonique, Tris, N-(2-acétamido)-2-acide iminodia-acétique, et des tampons préparés à partir de pyrophosphate, 4-(2-hydroxyéthyle)-1-piperazine éthane acide sulfonique (HEPES), acétate, imidazole, succinate, maléate, citrate, carbonate, sulfure de méthyle éthyle (MES), 3-(N-morpholino) acide propane sulfonique (MOPS), et des combinaisons de ceux-ci.

**8.** Un procédé selon l'une quelconque des Revendications précédentes, où l'échantillon contenant des globules sanguins demeure en contact avec la première composition pendant entre environ 10 à 40 minutes avant l'ajout de la deuxième composition à l'opération (ii).

**9.** Un procédé selon l'une quelconque des Revendications précédentes, où la concentration d'aldéhyde aliphatique dans la deuxième composition se situe entre 0,01 et 5% (v/v), de préférence entre 0,03 et 1 % (v/v), de préférence encore entre 0,05 et 0,5% (v/v), et idéalement entre 0,1 et 0,2% (v/v).

**10.** Un procédé selon l'une quelconque des Revendications précédentes, où la quantité totale d'aldéhyde aliphatique dans la composition combinée à l'opération (ii) se situe entre 0,05 et 0,5% (v/v), de préférence entre 0,1 et 0,2% (v/v).

**11.** Un procédé selon l'une quelconque des Revendications précédentes, où la concentration d'aldéhyde aliphatique dans la première composition est plus élevée que la concentration d'aldéhyde aliphatique dans la deuxième composition.

**12.** Un procédé selon la Revendication 11, où la concentration d'aldéhyde aliphatique dans la première composition est au moins le double de la concentration d'aldéhyde aliphatique dans la deuxième composition, de préférence au moins 5 fois supérieure, de préférence encore au moins 8 fois supérieure, et idéalement la concentration d'aldéhyde aliphatique dans la première composition est environ 10 fois supérieure à la concentration d'aldéhyde aliphatique dans la deuxième composition.

**13.** Un procédé selon l'une quelconque des Revendications précédentes, où l'agent chélateur dans la deuxième composition est sélectionné dans le groupe se composant d'acide éthylène diamine tétraacétique (EDTA), éthylène glycol-bis(β-amino éthyle éther)-*N,N,N',N'*-acide tétraacétique (EGTA), trans-1,2-diamino-cyclohexane-*N,N, N'N'*-acide tétraacétique (CDTA), acide nitriloacétique (NTA), porphine, hème, 1,2-bis(o-aminophénoxy)éthane-*N, N,N',N'*-acide tétraacétique (BAPTA), dimercaprol, et des combinaisons de ceux-ci.

**14.** Un procédé selon l'une quelconque des Revendications précédentes, où le conjugué de globule sanguin est un agrégat de plaquettes.

**15.** Un kit de stabilisation de conjugués de globules sanguins, le kit comprenant un premier récipient dans lequel se trouve une première composition contenant un aldéhyde aliphatique et un tampon, et un deuxième récipient dans lequel se trouve une deuxième composition contenant un aldéhyde aliphatique, un agent chélateur et un tampon, et éventuellement des instructions d'utilisation, où la concentration d'aldéhyde aliphatique dans la première composition est plus élevée que la concentration d'aldéhyde aliphatique dans la deuxième composition.

# Figure 1

Unstimulated blood

1a) 1b) 1c)

Stimulated blood

1d) 1e) 1f)

# EP 2 291 652 B1

## Figures 2a & 2b

2a) Determination of EDTA count

|        | Events | Aggregation |
|--------|--------|-------------|
| EDTA   | 1118   | 0 %         |

2b) Stimulant: Collagen 1μg/ml

|           | Events | Aggregation |
|-----------|--------|-------------|
| EDTA      | 1118   |             |
| Collagen  | 693    | 38 %        |

28

# Figures 2c & 2d

**2c) Stimulant: PAF 3μM**

| | Events | Aggregation |
|---|---|---|
| EDTA | 1118 | |
| PAF | 333 | **70 %** |

**2d) Stimulant: TRAP 10μM**

| | Events | Aggregation |
|---|---|---|
| EDTA | 1118 | |
| TRAP | 123 | **89 %** |

## Figures 2e & 2f

2e) Stimulant: <u>AA 200nM</u>

|        | Events | Aggregation |
|--------|--------|-------------|
| EDTA   | 1118   |             |
| AA     | 519    | **54 %**    |

2f) Stimulant: <u>U46619 1μM</u>

|        | Events | Aggregation |
|--------|--------|-------------|
| EDTA   | 1118   |             |
| U46619 | 43     | **96 %**    |

# Figure 3

## 3) Aggregation curve
### Stimulant: range of ADP

|  | Events | Aggregation |
|------|--------|-------------|
| EDTA | 1716 | 0 % |
| SAL | 1538 | 10 % |
| 1 ADP | 1259 | 27 % |
| 3 ADP | 658 | 62 % |
| 10 ADP | 182 | 89 % |

# Figure 4

## Figure 5

# Figure 6

## Figure 7

# Figure 8

## PLATELET AGGREGATION KIT

**A**

Citrate

1 volume of blood
to each aggregation tube

**STIR 4 MIN AT 37°C**

1 2 3 4 5

**B**

1 volume of Aggfix
to each aggregation tube

**WAIT 30 MIN**

1 2 3 4 5

**C**

1 2 3 4 5

1 volume of fixed blood
to each micro tube
containing Aggstabiliser

**CAP AND MIX**

1 2 3 4 5

## Figure 9

## Figure 10

**a) saline**

**b) collagen**

# Figure 11

## i) Platelet-Monocyte conjugates detected with CD42a

a) Saline

b) Collagen

## ii) Platelet-Monocyte conjugates detected with CD62P

a) Saline

b) Collagen

# Figure 12

### i) Platelet-Neutrophil conjugates detected with CD42a

a) Saline

b) Collagen

### ii) Platelet-Neutrophil conjugates detected with CD62P

a) Saline

b) Collagen

## Figure 13

a) Monocyte CD42a % gated

b) Monocyte CD42a mf

## Figure 14

a) Monocyte CD62P

b) Monocyte CD62P

## Figure 15

a) Neutrophil CD62P % gated

b) Neutrophil CD62P mf

## Figure 16

Aggregation measured before and after treatment with clopidogrel

Aggregation measured before and after treatment with clopidogrel and aspirin

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008107724 A
- US 6913932 B
- US 6872572 B
- US 20070166389 A1
- US 3925541 A
- US 5503982 A
- US 20040038424 A

**Non-patent literature cited in the description**

- **SCHMIDT V. ; HILBERG, T.** ThromboFix™ Platelet Stabilizer: Advances in clinical platelet analyses by flow cytometry?. *Platelets,* June 2006, vol. 17 (4), 266-273
- **ZOLA H et al.** CD molecules 2005: human cell differentiation molecules. *Blood,* 2005, vol. 106, 3123-3126